(19) Europäisches Patentamt
European Patent Office
Office européen des brevets

(11) Publication number: 0 034 883
A1

(12) # EUROPEAN PATENT APPLICATION

(21) Application number: 81300351.4

(22) Date of filing: 27.01.81

(51) Int. Cl.³: C 07 C 79/46
A 01 N 37/10, C 07 C 153/11
A 01 N 35/04, C 07 D 213/30
C 07 D 333/46, C 07 C 121/38
C 07 C 149/18, C 07 F 9/40
C 07 C 79/36, C 07 C 103/30

(30) Priority: 01.02.80 US 117732

(43) Date of publication of application: 02.09.81 Bulletin 81/35

(84) Designated Contracting States: AT BE CH DE FR IT LI NL SE

(71) Applicant: MOBIL OIL CORPORATION
150 East 42nd Street
New York New York 10017(US)

(72) Inventor: Theissen, Robert James
474 Van Holten Road
Bridgewater New Jersey 08807(US)

(74) Representative: Cooper, John Anthony et al,
Mobil Court 3 Clements Inn
London WC2A 2EB(GB)

(54) **2-Nitro-5-(substituted-phenoxy) benzoyl derivatives as herbicides.**

(57) Compounds of formula

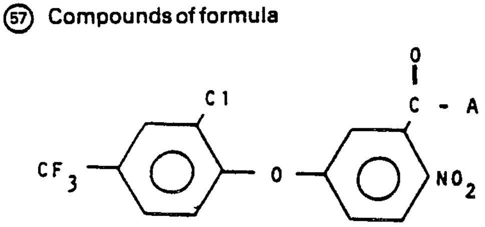

that have pre and post emergence activity. The substituent A is such that they include certain substituted phenyl esters, phenyl thioesters, heterocyclic esters; substituted alkyl and alkylthio esters, carbonates and thiocarbonates; benzoyl phosphonates; substituted alkanones and substituted amides.

EP 0 034 883 A1

# 2-NITRO-5-(SUBSTITUTED-PHENOXY) BENZOYL DERIVATIVES AS HERBICIDES

This invention is concerned with herbicidal 2-nitro-5-(substituted-phenoxy) benzoyl derivatives.

United States Patent Nos. 3,652,645; 3,784,635; 3,873,302; 3,983,168 and 3,907,866 describe certain phenoxy benzoic acid derivatives which are useful as herbicides. We have now found further phenoxy benzoic acid derivatives which are useful as herbicides.

This invention provides certain herbicidal compounds falling within the general formula:

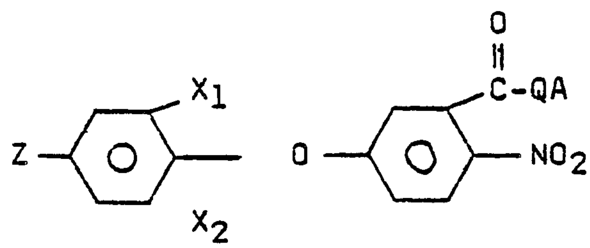

(I)

wherein $X_1$ is halogen, $X_2$ is selected from halogen and hydrogen, and Z is a polyhalo$_{1-9}$ alkyl$_{1-4}$ group such as $CF_3$, $CHF_2$, $C_4F_9$, $CF_2CH_2CH_3$ and $CH_2Cl$. Preferably, $X_1$ is chloro, Z is $CF_3$ and $X_2$ is hydrogen. QA in the formula includes certain groups more fully described below.

The specific 2-nitro-5-(substituted-phenoxy) benzoyl compounds are described below. One method for preparing these compounds is the use of the Ullmann ether synthesis reaction between the alkali metal (e.g., Na, K) salt of a suitable substituted phenol, e.g., m-hydroxy benzoic acid or m-cresol with an active halogen-substituted aromatic, e.g., 3,4-dichlorobenzotrifluoride. The intermediate obtained may be nitrated and the desired final derivatives subsequently prepared by known procedures to the desired benzoyl compound. Where m-cresol is used as a starting material, the

product obtained can be oxidized and subsequently nitrated before the derivative is prepared.

A. Substituted phenyl esters, phenylthio ester, substituted phenylthio esters

Illustrative compounds of this class are those of the formula:

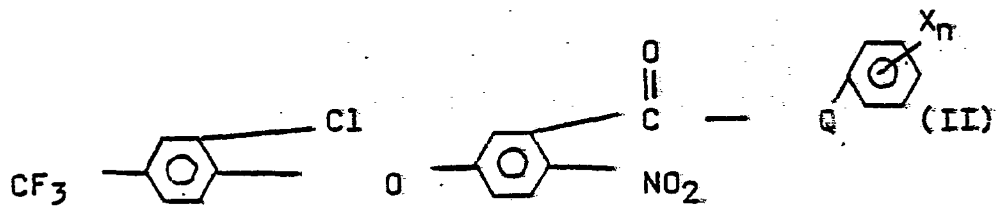

(II)

Where Q is O or S, n is an integer from 1 to 5, and X is selected from $NO_2$; lower alkyl of $C_{1-4}$; halogen; phenyl; COOR where R is lower alkyl of $C_{1-4}$, H or a cation selected from alkali metals, alkaline earth metals, ammonium and lower alkyl and di-lower alkyl ammonium; CN; lower alkyl thio of $C_{1-4}$; lower alkoxy of $C_{1-4}$; trifluoromethyl; and combinations thereof. X can be hydrogen when Q is sulfur.

Compound 1

Preparation of 2,4-dinitro-6-sec-butylphenyl 5-[-2 chloro-4-(trifluoromethyl) phenoxy]-2-nitrobenzoate.

To a stirred solution of 2,4-dinitro-6-sec-butylphenyl (3.60 g, 0.015 mole) and 5-[-2-chloro-4-(trifluoromethyl) phenoxy]-2-nitrobenzoyl chloride (5.68 g, 0.015 mole) in toluene (25 ml) was added triethylamine (1.50 g, 0.015 mole). The temperature exothermed to 45°C and there was an immediate precipitation of triethylamine hydrochloride. The reaction was stirred and heated to 110°F for 15 hours. The precipitate was filtered from the cooled solution, washed and dried to give triethylamine hydrochloride (1.8 g). The toluene solution was washed with 10% sodium hydroxide (3 x 25 ml). The toluene was then stripped off a rotary evaporator to give 5.5 g (62%)

of a crude brown oil which slowly crystallized. Recrystallization from methanol gave 2.1 g of an off white solid, m.p. 109 - 110°C. I.R. (nujol): C = O, 1765 $cm^{-1}$

NMR ($D_6$ Acetone): triplet 0.9 ppm (3H, J = 3.5 Hz):
doublet 1.37 ppm (3H,J = 3.5 Hz):
multiplet 1.82 ppm (2H)
multiplet 3.35 ppm (1H)
multiplet 7.5 - 8.2 (5H)
doublet 8.45 ppm (1H, J = 4.5 Hz);
doublet 8.82 ppm (1H, J = 1.2 Hz);
doublet 9.02 ppm (1H, J = 1.2 Hz).

In a similar manner the following com- pounds were prepared as defined according to Formula II:

| Compound | Q (O or S) | Phenyl Substitution $X_n$ | m.p., °C |
|---|---|---|---|
| 2 | O | $2-NO_2$ | 137-9 |
| 3 | O | $3-NO_2$ | 93.5 |
| 4 | O | $4-NO_2$ | 115-7 |
| 5 | O | $5-CH_3-2NO_2$ | 88-90 |
| 6 | O | $2-Cl-4NO_2$ | 99-101 |
| 7 | O | 4-Cl | 135-7 |
| 8 | O | $4-Cl-2CH_3$ | 121-2 |
| 9 | O | $2-Cl-4-CH_3$ | 105-7 |
| 10 | O | 2-Cl-4-phenyl | 128-30 |
| 11 | O | $2,4-(Cl)_2$ | 144-7 |
| 12 | O | $4-COOCH_3$ | 103-6 |
| 13 | O | 4-CN | 103-5 |
| 14 | O | $4-SCH_3$ | 94-5 |
| 15 | O | $3-OCH_3$ | 95-7 |
| 16 | O | $4-OCH_3$ | 128-9 |
| 17 | O | $3-CF_3$ | oil |
| 18 | O | 4-COONa | 215-20 |
| 19 | O | 4-COOH | 191-8 |
| 20 | S | H | oil |
| 21 | S | 4-Cl | 128-30 |
| 22 | S | $4-CH_3$ | 90-3 |
| 23 | S | $4-OCH_3$ | oil |
| 24 | S | $3,4-(Cl)_2$ | oil |

Other compounds of this class include:

Compound

2,6-diethylphenyl 5-[2-chloro-4-(trifluoromethyl) phenoxy]-2-nitrobenzoate

S-[2-carboxyphenyl) 5-[2-chloro-4-(trifluoromethyl) phenoxy]-2-nitrobenzenecarbothioate

4-cyano-2,6-dibromophenyl 5-[2-chloro-4-(trifluoromethyl) phenoxy]-2-nitrobenzoate (m.p. 170-1°C I.R. C = O $1775 cm^{-1}$)

3,6-dichloro-2-methoxycarbonyl-phenyl 5-[2-chloro-4-(trifluoromethyl) phenoxy]-2-nitrobenzoate

(2,6-di-6-butyl)phenyl 5-[2-chloro-4-(trifluoromethyl) phenoxy]-2-nitrobenzoate (oil I.R. C = O $1752 cm^{-1}$

pentachlorophenyl 5-[2-chloro-4-(trifluoromethyl) phenoxy]-2-nitrobenzoate

B. Heterocyclic Esters

Illustrative compounds of this class are those of the formula:

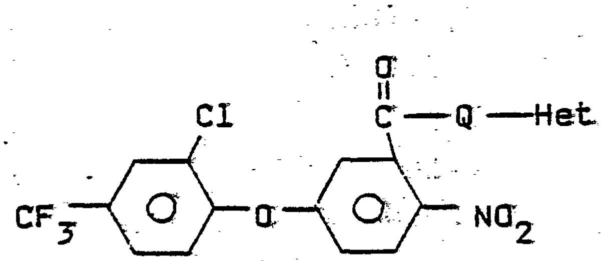

(III)

where Q is O or S and Het is a substituted or unsubstituted heterocyclic ring of 5 to 7 members comprising carbon in combination with one or more of sulfur, oxygen and nitrogen where a carbon atom on the ring is bound directly to Q.

Compound 25

Preparation of 3-pyridyl 5-[2-chloro-4-(trifluoromethyl) phenoxy]-2-nitrobenzoate: To a stirred solution of 5-[2-chloro-4-(trifluoromethyl) phenoxy]-2-nitrobenzoyl chloride (3.79g, 0.01 mole) and triethyl amine (1.01g, 0.01 mole) in toluene (50 ml) was added 3-hydroxypyridine (0.95g, 0.01 mole). The temperature exothermed to 33$^{o}$ and there was an immediate precipitate. The reaction was then refluxed for 24 hours, cooled to room temperature and the triethylamine hydrochloride precipitate collected by filtration. The toluene solution was washed with dilute sodium hydroxide solution and then with saturated sodium chloride solution. After drying and evaporation of the solvent there was obtained 3.1g of brown oil which solidified. This was triturated with hexane and the solid filtered and dried to give a tan product, m.p. 65-68$^{o}$C.

I.R. (nujol): C = O, 1765 $cm^{-1}$

NMR($D_6$-acetone): complex multiplet 7.2-8.1 ppm (7H); doublet 8.50 ppm (1H, J = 4.5Hz); multiplet 8.75 ppm (2H)

Compound 26

This compound is defined in accordance with Formula III as "Q" is O and "Het" is 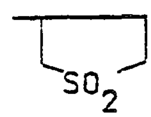 .

The compound was prepared by the procedure shown for Compound 25. Compound 26 has an m.p of 153 - 161°C.

Other compounds of this class include:

Compound

3-(1,2,5 thiadiazoloyl)-5-[2-chloro -4-trifluoromethyl) phenoxy]-2-nitrobenzoate

1,2-dihydro-1,6-dimethyl-2-oxo 4-pyridinyl 5-[2-chloro-4-(trifluoromethyl) phenoxy] -2-nitrobenzoate

6(2,3-dihydro-3-oxo-pyridazinyl)
5-[2-chloro-4-(trifluoromethyl)
phenoxy]-2-nitrobenzoate

2-chloro-6-pyridyl 5-[2-chloro-
4-(trifluoromethyl) phenoxy]-2-
nitrobenzoate

s-(2,6-dichloro-4-s-triazinyl)
5-[2-chloro-4-(trifluoromethyl)
phenoxy]-2-nitrobenzoate

S-(2-methyl 1,3,4,-thiadiazol-5-yl)
5-[2-chloro-4-(trifluoromethyl)
phenoxy]-2-nitrobenzenecarbothioate

S-(2-pyridinyl)5-[2-chloro-4-
(trifluoromethyl) phenoxy]-2-
nitrobenzenecarbothioate
N-oxide

S-[4-methyl-3-thioxo-3H-1,2-dithiol
-5-yl] 5-[2-chloro-4-(trifluoro-
methyl) phenoxy]-2-nitrobenzene-
carbothioate

S-[3,5-bis-(diethylamino)-s-tri-
azinyl] 5-[2-chloro-4-(trifluoro-
methyl) phenoxy]-2-nitrobenzene-
carbothioate

O-[2-oxo-furan-3-yl-]
5-[2-chloro-4-(trifluromethyl)
phenoxy]-2-nitrobenzene carboxylate
(oil, I.R. C = O 1797 and 1750 $cm^{-1}$)

C. Substituted $C_{-1-4}$ Alkyl Esters

Illustrative compounds of this class are those of the formula:

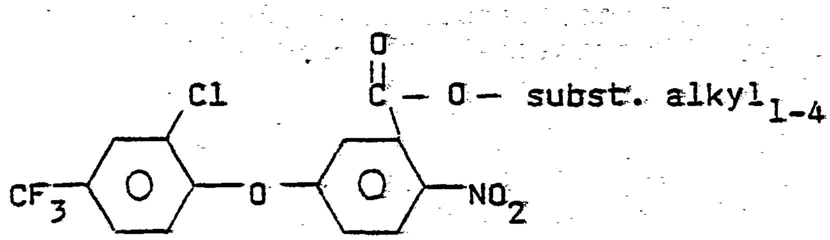

(IV)

wherein the substituents on the straight or branched $C_{1-4}$ alkyl group are selected from cyano; phenyl;

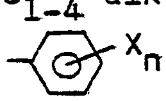 where n is 1 to 5 and X is selected from $NO_2$,

lower alkyl of $C_{1-4}$, halogen, phenyl, phenoxy, CN, lower alkyl thio of $C_{1-4}$, lower alkoxy of $C_{1-4}$, trifluoromethyl and combinations thereof; $-\overset{O}{\overset{\|}{C}}-R_2$ ; $-O-\overset{O}{\overset{\|}{C}}-R_2$; phenoxy; nitro; $SR_1$; $SOR_1$; $SO_2R_1$; where $R_1$ is an alkyl of 1 to 3 carbons and $R_2$ is an alkyl or alkenyl of up to 3 carbons and wherein one or more of said substituents can be attached to any one or more of the carbons on said straight or branched $C_{1-4}$ alkyl group.

The compounds of this class may be prepared by the methods shown above or alternatively by displacement of an active halogen (e.g., Haloalkyl X) with the salt of an acid (e.g., $AR-\overset{O}{\overset{\|}{C}}-OK$).

The following substituted $C_{1-4}$ alkyl esters as defined by Formula IV were prepared:

| Compound | Substituted Alkyl Group | m.p.°C |
|---|---|---|
| 27 | $-C(CH_3)_2CN$ | oil |
| 28 | $-CH(CH_3)CN$ | oil |
| 29 | $-CH_2COCH_3$ | 83-5 |
| 30 | 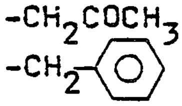 | oil |
| 31 | 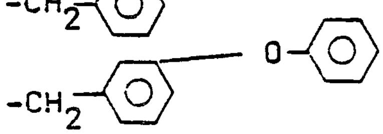 | oil |
| 32 | $-CH_2-CH_2-O\overset{O}{\overset{\|}{C}}-CH=CH_2$ | oil |
| 33 | $-CH_2-CH_2-O\overset{O}{\overset{\|}{C}}-\overset{CH_3}{\overset{\vert}{C}}=CH_2$ | oil |
| 34 | $-CH_2-CH_2-CN$ | oil |
| 35 | | |
| 36 | 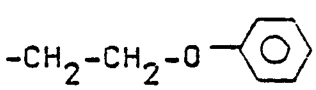 | 86-8 |
| 37 | 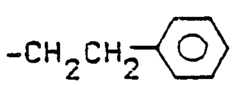 | 112-3 |

| | | |
|---|---|---|
| 38 | $-CH_2C(CH_3)_2-NO_2$ | oily semisolid I.R. C=O 1740 $cm^{-1}$ |
| 39 | $-CH_2-CH_2-SCH_3$ | oil |
| 40 | $-CH_2-CH_2-SO_2CH_3$ | 97-9 |

Other compounds of this class include:

| | |
|---|---|
| $-CH_2CN$ | oil, I.R. C=O 1725 $cm^{-1}$ |
| $-CH_2-C(OCH_3)_2-CH_3$ | m.p. 73-4° |
| $-CH_2-CH(CH_3)-NO_2$ | oil, I.R. C=O 1740 $cm^{-1}$ |
| $-CH_2-CH(OC_2H_5)_2$ | 1740 $cm^{-1}$ |
| $-CH_2-SO-CH_3$ | 115-8° I.R. C=O 1745 $cm^{-1}$ |
| $-CH_2-CH_2-SO-CH_3$ | 113-5° I.R. C=O 1745 $cm^{-1}$ |
| $-CH_2-S-CH_3$ | oil I.R. C=O 1740 $cm^{-1}$ |

| Substituted alkyl | Compound Name |
|---|---|
| $CH_2-S-CH_2CH_3$ | (ethylthio)methyl 5-[2-chloro-4-(trifluoromethyl) phenoxy]-2-nitro-benzoate |
| 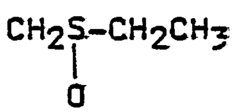 | (ethylsulfinyl)methyl 5-[2-chloro-4-(trifluoromethyl) phenoxy]-2-nitro-benzoate |
| 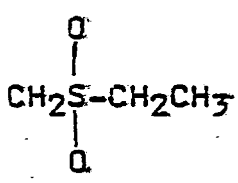 | (ethylsulfonyl)methyl 5-[2-chloro-4-(trifluoromethyl) phenoxy]-2-nitrobenzoate |
| 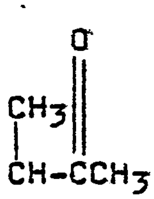 | 1-acetylethyl 5-[2-chloro-4-(trifluoromethyl) phenoxy]-2-nitro-benzoate |
| 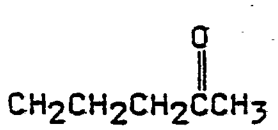 | 3-acetylpropyl 5-[2-chloro-4-(trifluoromethyl) phenoxy]-2-nitrobenzoate |

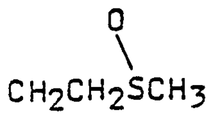

2-(methylsulfinyl)ethyl 5-[2-chloro-4-(trifluoromethyl) phenoxy]-2-nitrobenzoate

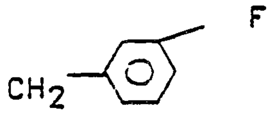

(3-fluorophenyl) methyl 5-[2-chloro-4-(trifluoromethyl) phenoxy]-2-nitrobenzoate (oil, I.R. C=O 1735 $cm^{-1}$

D. <u>Carbonates and Thiocarbonates</u>

Illustrative compounds of this class are those of the formula:

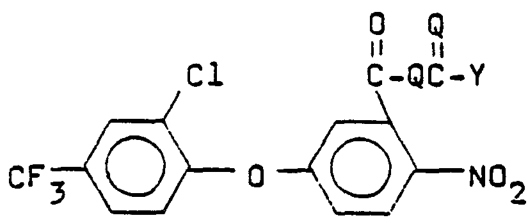

(V)

where Q is O or S and Y is a straight or branched alkoxy, alkylthio, alkenyloxy or alkenylthio of 1 to 6 carbons, a dialkylamino of 2 to 6 carbons, or a heterocyclic amine such as morpholine.

<u>Compound 41</u>

Preparation of S[(ethylthio) carbonyl] 5-[2-chloro-4-(trifluoromethyl) phenoxy]-2-nitrobenzoate.

To a stirred solution of 5-[2-chloro-4-(trifluoromethyl) phenoxy]-2-nitrobenzoic acid (3.0g, 0.0083 mole) in ether (15 ml) was added triethylamine (0.84g, 0.0083 mole). A precipitate formed. The reaction was cooled to 4°C and a solution of ethylchlorothioformate (1.03 g, 0.0083 mole) in ether was added dropwise. Cooling was maintained during initial exotherm. The reaction was then stirred at room temperature for twenty hours. The precipitate of triethyl amine hydrochloride was filtered. The ether was washed consecutively with 10% hydrochloric acid solution, 5% sodium hydroxide solution and water. The dried solution was evaporated to give 3.04 g of a yellow oil.

I.R. (neat): C = O, 1675 and 1800 $cm^{-1}$
NMR ($CDCl_3$): triplet 1.37 ppm (3H, J = 7.5 Hz);
quartet 3.12 ppm (2H, J = 7.5 Hz);
complex multiplet 7.0 - 8.0 ppm (5H);
doublet 8.15 ppm (1H, J = 4.8 Hz).

In a similar manner, the following compounds as defined in accordance with formula V, were prepared:

| Compound | Q | Y | m.p., °C |
|---|---|---|---|
| 42 | O | $OCH_3$ | oil |
| 43 | O | $OCH_2CH_3$ | oil |
| 44 | O | $OCH_2CH_2CH_3$ | oily semi-solid |
| 45 | O | $OCH(CH_3)_2$ | oily semi-solid |
| 46 | O | $OCH_2CH_2CH_2CH_3$ | oil |
| 47 | O | $OCH_2CH(CH_3)_2$ | oil |
| 48 | S | $OCH_3$ | oil |
| 49 | S | morpholino | 137-8[a] |

S[(propylthio) thionocarbonyl] 5-[2-chloro-4-(trifluoromethyl) phenoxy]-2-nitrobenzenecarbothioate is a further illustrative compound of this class wherein Q is S and Y is $-SC_3H_7$, as are the following compounds:

| Q | Y | m.p., °C/I.R. $cm^{-1}$ |
|---|---|---|
| S | $OC_2H_5$ | oil, 1670 |
| S | $O-CH_2-CH=CH_2$ | oil, 1672 |
| S | $OC_3H_7$ | oil, 1668 |
| S | $OCH(CH_3)_2$ | oil, 1740-1700 |

E. Benzoyl Phosphonates

Illustrative compounds of this class are those of the formula:

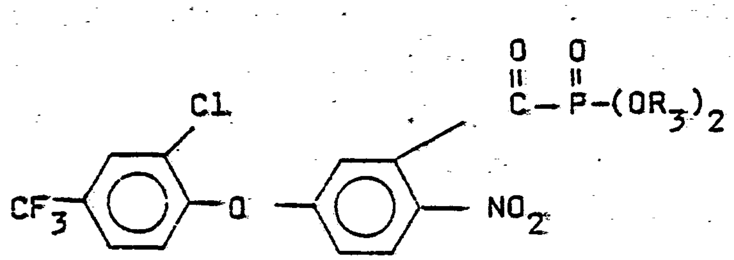

(VI)

where $R_3$ is straight or branched alkyl of 1 to 6 carbons.

Compound 50

Preparation of 5-[2-chloro-4-(trifluoromethyl) phenoxy]-2-nitrobenzoyl-phosphonic acid, diisopropyl ester.

To a stirred solution of 5-[2-chloro-4-(trifluoromethyl) phenoxy]-2-nitrobenzoyl chloride (3.8g, 0.01 mole) in ether (100 ml) was added triisopropyl phosphite (2.1g, 0.01 mole). The reaction was refluxed for one hour and the disappearance of phosphite monitored by v.p.c. The ether was stripped off and the resulting oil placed under vacuum (1.0 mm) at 40°C. for two hours. The resulting amber liquid amounted to 5.2g.

I.R. (neat): C = O, 1692 and 1730 $cm^{-1}$

NMR ($CDCl_3$): doublet 1.29 ppm (3H, J = 3.0 Hz);
doublet 1.37 ppm (3H, J = 3.0 Hz);
multiplet 4.88 ppm (1H);
complex multiplet 6.9 - 8.0 ppm (5H);
broad doublet 8.37 ppm (1H, J = 4.5 Hz).

In a similar manner, the following compound was prepared as an additional example of a benzoyl phosphonate.

Compound 51

5-2[-chloro-4-(trifluoromethyl) phenoxy]-2-nitrobenzoyl-phosphonic acid diethyl ester was prepared and was an oil.

F. Substituted Alkanone Compounds

Illustrative compounds of this class are those of the formula:

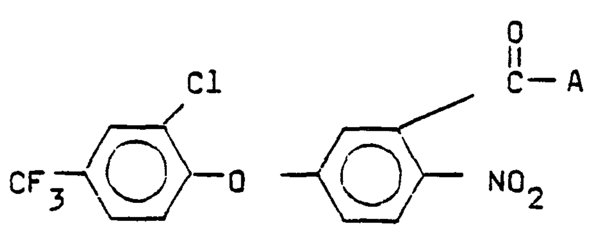

(VII)

where A is an alkyl of 1 to 3 carbons or hydrogen.

Compound 52

Preparation of 5-[2-chloro-4-(trifluoromethyl) phenoxy]-2-nitrophenyl ethanone (A is $-CH_3$).

A stirred solution of 3-[2-chloro-4-(trifluoromethyl) phenoxy] phenyl ethanone (15.7 g, 0.05 mole) in dichloroethane (40 ml) was cooled to 0-5°C. Nitric acid (95%) (4.95 g, 0.075 mole) was added, followed by the dropwise addition of 95% sulfuric acid (12.3 g, 0.125 mole) while maintaining the temperature below 5-10°C. Allowed reaction to warm to room temperature. The progress of the reaction was monitored by v.p.c. The reaction solution was poured onto ice water and the organic solution separated, washed with water and dried. The solvent was evaporated to give 16 g of a brown oil, which was extracted with hot heptane. Upon cooling, the heptane solution furnished an oil fraction which solidified to give 8.8 g of a brown solid, m.p. 58-60°C. I.R. (nujol mull): C = O, 1710 $cm^{-1}$; $NO_2$, 1530 and 1315 $cm^{-1}$.

Compound 53

5-[2-chloro-4-trifluoromethyl)phenoxy]-2-nitrobenzaldehyde ("A" is hydrogen in Formula VII) was prepared. It had a melting point of 99-100°C.

G. Amides

Illustrative compounds of this class include those of the formula:

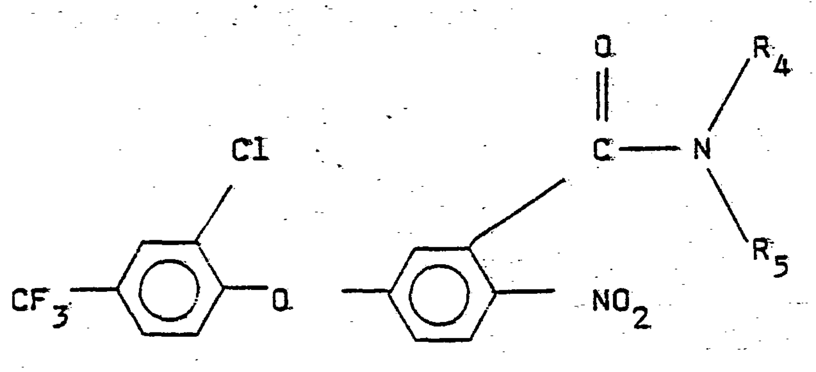

(VIII)

wherein $R_4$ is selected from hydrogen and straight or branched chain alkyl of 1-4 carbon; substituted or unsub-

stituted; $R_5$ is selected from: phenyl; 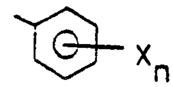
(where n is 1 to 5 and X is selected from $NO_2$, lower alkyl of $C_{1-4}$, halogen, phenyl or phenoxy), COOR (where R is lower alkyl of $C_{1-4}$ or H or a cation), CN, lower alkyl thio of $C_{1-4}$, lower alkoxy of $C_{1-4}$, trifluoromethyl and combinations thereof; heterocyclicalkyl and heterocyclic, said heterocyclics of a 5 to 7 member ring comprising carbon in combination with one or more of sulfur, oxygen and nitrogen; hydroxyl, alkoxy of 1-3 carbons; $-R_6CO_2R_6$, where $R_6$ is an alkyl or alkenyl of up to 3 carbons; $-R_6CONH_2$; $-R_6CONHR_2$ or $R_6CON(R_2)_2$; (where $R_2$ is as defined above) alkoxyalkyl where the alkoxy and alkyl may each contain a straight or branched $C_{1-3}$ alkyl chain; or where $R_4$ and $R_5$ together form a heterocyclic ring as hereinabove defined.

<u>Compound 54</u>

Preparation of N-phenyl 5-[2-chloro-4-(trifluoromethyl) phenoxyl]-2-nitrobenzamide:

To a stirred solution of 5-[2-chloro-4-(trifluoromethyl) phenoxy]-2-nitrobenzoyl chloride (5.68 g, 0.015 mole) in toluene (35 ml) was added aniline (2.8 g, 0.30 mole). The temperature immediately rose to 45°C. The reaction was then heated to reflux for twelve hours. The cooled reaction was poured into dilute hydrochloric acid solution. The residual solids were filtered, washed with warm water and dried to give 4.1 g of a gray solid m.p. 190-4°C. Recrystallization from methanol gave 3.8g, m.p. 191-4°C.

I.R. (nujol): C = O, 1660 $cm^{-1}$

NMR ($D_6$-Acetone): complex multiplet 7.0 - 8.1 ppm (10H); doublet 8.32 ppm (1H, J = 4.5 Hz); broad singlet 9.95 ppm (1H).

In a similar manner, the following compounds were prepared:

| Example | $R_4$ | $R_5$ | m.p., °C/oil |
|---|---|---|---|
| 55 | H | 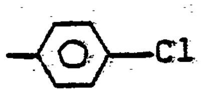 | 145-8 |
| 56 | H | 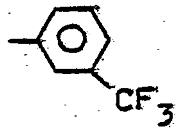 | 179-81 |
| 57 | H | 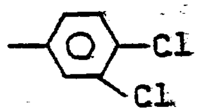 | 155-8 |
| 58 | H | 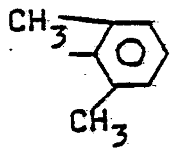 | 169-71 |
| 59 | H | 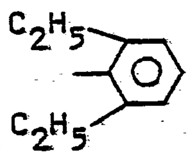 | 185-7 |
| 60 | $CH(CH_3)_2$ | 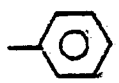 | 144-6 |
| 61 | H | 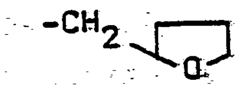 | 140-1 |
| 62 | H | OH | oil |
| 63 | H | $OCH_3$ | 171-4 |
| 64 | $CH_3$ | OH | oil |
| 65 | $CH_3$ | $OCH_3$ | oil |
| 66 | H | $CH_2COOC_2H_5$ | 136-7 |
| 67 | H | $CH_3$<br>$-CHCOOC_2H_5$ | oil |
| 68 | H | $CH_3$<br>$-CHCONH_2$ | 160-3 |
| 69 | H | $-CH_2CH_2OC_2H_5$ | 86-8 |
| 70 | H | $-CH_2CH_2CH_2OCH_3$ | 102-4 |
| 71 | H | 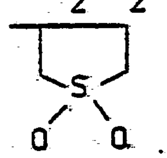 | 158-60 |

| Compound | $R_4$ and $R_5$ | m.p. °C/oil 112 |
|---|---|---|
| 72 | 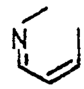 | 107-8 |
| 73 | 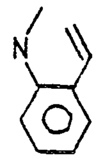 | 128-32 |
| 74 | 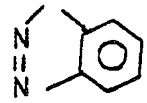 | 124-6 |
| 75 | 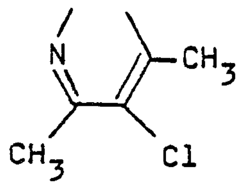 | 169-72 |
| 76 | 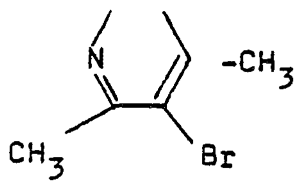 | oil |
| 77 | 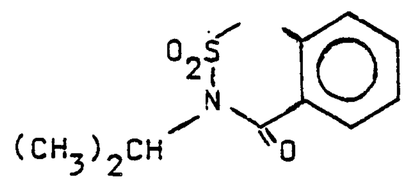 | 125-8 |

Other illustrative compounds of this class include:

Compound Name

N-[2-(5-t-butyl-1,3,4 thiadia zolyl)] 5-[2-chloro-4-(trifluoromethyl) phenoxy]-2-nitrobenzamide

5-[2-chloro-4-(trifluoromethyl phenoxy]-2-nitrobenzoyl 2,5-di xo-pyrolidine

N-[2-(4,6,6-trimethyl-1,3-thiazinyl)] 5-[2-chloro-4-(tri-fluoromethyl) phenoxy]-2-nitro benzamide

N-(carboxymethyl) 5-[2-chloro-4-(trifluoromethyl) phenoxy]-2-nitro benzamide

N-carboxymethyl-N-methyl 5-[2-chloro-4-(trifluoromethyl) phenoxy]-2-nitrobenzamide

N-(2,5-dichloro-3-methoxycarbonyl) phenyl 5-[2-chloro-4-(trifluoromethyl) phenoxy]-2-nitrobenzamide

N-[4-[6-(1,1-dimethylethyl)-3-methylthio-1,2,4-triazin-5(4H)-onyl]] 5-[2-chloro-4-(trifluoromethyl) phenoxy]-2-nitrobenzamide

N-(3,4-dimethyl-2,6-dinitrophenyl) -N-(1-methylethyl) 5-[2-chloro-4-(trifluoromethyl) phenoxy]-2-nitrobenzamide

N-[2,6-dinitro-4-(trifluoromethyl) phenyl]-N-ethyl 5-[2-chloro-4-(trifluoromethyl) phenoxy]-2-nitrobenzamide

Further compounds are those in which the $NR_4R_5$ group is as follows:

| | |
|---|---|
| $-NHCOOCH(CH_3)_2$ | m.p. 176-9°, I.R. C=O 1760 and 1680 $cm^{-1}$ |
| $-NHCOOC_2H_5$ | m.p. 155-6°, I.R. C=O 1775 and 1692 $cm^{-1}$ |
| $-NHCOOC_3H_7$ | m.p. 171-30°, I.R. C=O 1765 and 1680 $cm^{-1}$ |
| $N(CH_2CH_2OH)$ 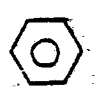 | m.p. 96-8°, I.R. C=O 1635 $cm^{-1}$ |
| $NH\ C(CH_3)_2CN$ | m.p. 63°, I.R. C=O 1655 $cm^{-1}$ |

| | |
|---|---|
| NHCN | m.p. 100-2°, I.R. C=O 1700 $cm^{-1}$ (very broad) |
| 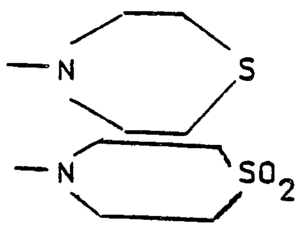 | oil I.R. C=O 1640 $cm^{-1}$<br>oil I.R. C=O 1655 $cm^{-1}$ |
| 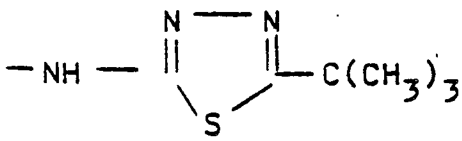 | 207-9°, I.R. C=O 1690 $cm^{-1}$ |
| $NHCH_2COONa$ | 58-60° I.R. C=O 1720 and 1655 $cm^{-1}$ |

H. Other Benzoyl Compounds

The following 5-[2-chloro-4-(trifluoromethyl) phenoxy] 2-nitro-benzoyl compounds do not readily fall within the defined classes A - G above and are therefore listed below:

Compound 78

2-chloro-cyclohexyl 5-[2-chloro-4-(trifluoromethyl)phenoxy]-2-nitrobenzoate (oil).

Compound 79

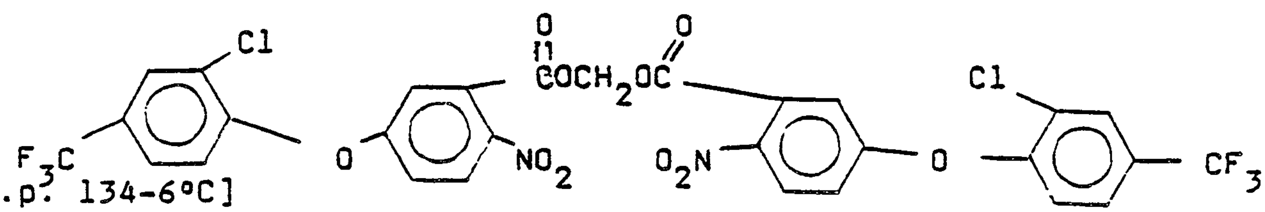

[m.p. 134-6°C]

Compound 80

S-(4-chloro-benzyl) 5-[(2-chloro-4-trifluoromethyl) phenoxy]-2-nitro-benzene carbothioate. [oil]

Compound 81

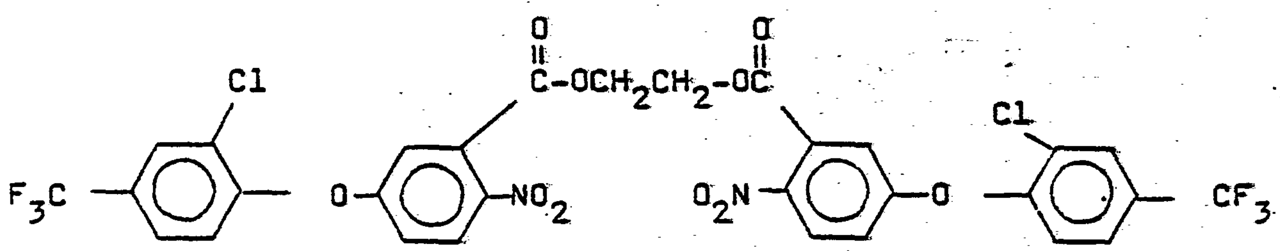

[oil]

Additional illustrative compounds are listed below:

4-(2,4-difluorophenyl)-2-(methoxycarbonyl) phenyl 5-[2-chloro-4-(trifluoromethyl) phenoxy]-2-nitrobenzoate

4-(ethoxycarbonylethyl)-2-hydroxyphenyl 5-[2 chloro-4-(trifluoromethyl) phenoxy]-2-nitrobenzoate

3,5-dinitro-4-dipropylaminophenyl 5-[2-chloro-4-(trifluoromethyl) phenoxy]-2-nitrobenzoate

2,6-bis(hydroxymethyl)-4-methylphenyl 5-[2-chloro-4-(trifluoromethyl) phenoxy]-2-nitrobenzoate

3-(1,2,5,6-diisopropylidene-D-glucosyl) 5-[2-chloro-4-(trifluoromethyl) phenoxy]-2-nitrobenzoate

2-chloro-9-(methoxycarbonyl) 9H-fluoren-9-yl 5-[2-chloro-4-(trifluoromethyl) phenoxy]-2-nitrobenzoate

-benzoyl (phenylmethyl) 5-[2-chloro-4-(trifluoromethyl) phenoxy]-2-nitrobenzoate

2-(acetylamino) ethyl 5-[2-chloro-4-(trifluoromethyl) phenoxy]-2-nitrobenzoate

2-(2-methylpropenoylamino) ethyl 5-[2-chloro-4-(trifluoromethyl) phenoxy]-2-nitrobenzoate

[3-(dimethylamino)-2-methyl propionyloxy] ethyl 5-[2-chloro-4-(trifluoromethyl) phenoxy]-2-nitrobenzoate

2-(2-methylpropenoyloxy)-2-methylethyl 5-[2-chloro-4-(trifluoromethyl) phenoxy]-2-nitrobenzoate

(1-cyano-1 methyl) ethoxymethyl 5-[2-chloro-4-(trifluoromethyl) phenoxy]-2-nitrobenzoate

(ethoxycarbonyl) (acetyl) methyl 5-[2-chloro-4-(trifluoromethyl) phenoxy]-2-nitrobenzoate

diethoxyphosphonylmethyl 5-[2-chloro-4-(trifluoromethyl) phenoxy]-2-nitrobenzoate

dimethoxyphosphinylethyl 5-[2-chloro-4-(trifluoromethyl) phenoxy]-2-nitrobenzoate

dimethoxyphosphinylethoxy ethyl 5-[2-chloro-4-(trifluoromethyl) phenoxy]-2-nitrobenzoate

2-(chloroacetyl) ethyl 5-[2-chloro-4-(trifluoromethyl) phenoxy]-2-nitrobenzoate

difluoromethyl 5-[2-chloro-4-(trifluoromethyl) phenoxy]-2-nitrobenzoate

propenoylaminomethyl 5-[2-chloro-4-(trifluoromethyl) phenoxy]-2-nitrobenzoate

cyano (ethoxycarbonyl) methyl 5-[2-chloro-4-(trifluoromethyl) phenoxy]-2-nitrobenzoate

4-(3-chlorophenylcarbamoyloxy) butyn-2-yl 5-[2-chloro-4-(trifluoromethyl) phenoxy]-2-nitrobenzoate

N-(Chloroacetyl)-N-(2,6-diethylphenylamino) methyl 5-(2-chloro-4-(trifluoromethyl) phenoxy]-2-nitrobenzoate

3-cyanopropen-2-yl 5-[2-chloro-4-(trifluoromethyl) phenoxy]-2-nitrobenzoate

5-[2-chloro-4-(trifluoromethyl) phenoxy]-2-nitrobenzoic acid anhydride with thiocyanic acid

S-(phenylsulfonylaminoethyl) 5-[2-chloro-4-(trifluoromethyl) phenoxy] 2-nitrobenzenecarbothioate

S(2,3-Dihydroxypropyl) 5-[2-chloro-4-(trifluoromethyl) phenoxy]-2-nitrobenzenecarbothioate

S-(ethoxycarbonylmethyl) 5-[2-chloro-4-(trifluoromethyl) phenoxy]-2-nitrobenzenecarbothioate

S-(carboxymethyl) 5-[2-chloro-4-(trifluoromethyl) phenoxy]-2-nitrobenzenecarbothioate

S-[(3-fluorophenyl) methyl] 5-[2-chloro-4-(trifluoromethyl) phenoxy]-2-nitrobenzenecarbothioate

S-[(ethylthio carbonyl) methyl] 5-[2-chloro-4-(trifluoromethyl) phenoxy]-2-nitrobenzenecarbothioate

S-[(2,3-dihydroxypropoxycarbonyl) methyl] 5-[2-chloro-4-(trifluoromethyl) phenoxy]-2-nitrobenzenecarbothioate

S-[(3-ethoxycarbonyl) propen-2-yl] 5-[2-chloro-4-(trifluoromethyl) phenoxy]-2-nitrobenzenecarbothioate

1,1 dimethyl-2,2,2-trichloroethoxycarbonyl 5-[2-chloro-4-(trifluoromethyl) phenoxy]-2-nitrobenzoate

5-[2-chloro-4-(trifluoromethyl) phenoxy]-2-nitrobenzoyl O-ethylphosphonic acid ammonium salt

1- 5-[2-chloro-4-(trifluoromethyl) phenoxy]-2-nitrophenyl 2,2,2,-trichloroethanone

1- 5-[2-chloro-4-(trifluoromethyl) phenoxy]-2-nitrophenyl 2,2,2,-trifluoroethanone

dimethyl 5-[2-chloro-4-trifluoromethyl) phenoxy]-2-nitrobenzoyl-methylphosphonate

5-[2-chloro-4-(trifluoromethyl) phenoxy] 2-nitrophenyl 4-methoxy-3-methyl methanone

1- 5-[2-chloro-4-(trifluoromethyl) phenoxy]-2-nitrophenyl 2-(di-methoxyphosphinyl methyl-amino) ethanone

5-[2-chloro-4-(trifluoromethyl) phenoxy]-2-nitrobenzoyl cyanade

N-(2,2-dimethoxyethyl) 5-[2-chloro-4-(trifluoromethyl) phenoxy]-2-nitrobenzamide

N-(carboxymethyl)-N-(phosphonomethyl) 5-[2-chloro-4-(trifluoromethyl) phenoxy]-2-nitrobenzamide

N-(carboxymethoxy) 5-[2-chloro-4-(trifluoromethyl) phenoxy]-2-nitro benzamide

N-(1-methylethyl)-N-phenylmethyl)
5-[2-chloro-4-(trifluoromethyl)
phenoxy]-2-nitrobenzamide

3 -5-[2-chloro-4-(trifluoromethyl)
phenoxy]-2-nitrobenzoyl 4-chloro
-2-oxobenzothiazoline

N-[1,3-dihydroxy-2-methylpropyl)
5-[2-chloro-4-(trifluoromethyl)
phenoxy]-2-nitrobenzamide

N-(diethoxyphosphinyl) 5-[2-chloro-
-4-(trifluoromethyl) phenoxy]-2-
nitrobenzamide

N-(2-sulfoethyl) 5-[2-chloro-4-
(trifluoromethyl) phenoxy]-2-
nitrobenzamide sodium salt

N-(2-hexyloxysulfonyl) ethyl 5-[2-
chloro-4-(trifluoromethyl) phenoxy]
-2-nitrobenzamide

N-(trifluoroacetyl) 5-[2-chloro-
4-(trifluoromethyl) phenoxy]-2-
nitrobenzamide

N-(4-sulfophenyl) 5-[2-chloro-
4-(trifluoromethyl) phenoxy]-2-
nitrobenzamide

N-[(1-cyano-1-methyl)-2-ethyl]
5-[2-chloro-4-(trifluoromethyl)
phenoxy]-2-nitrobenzamide

N-(ethoxycarbonyl) 5-[2-chloro-
4-(trifluoromethyl) phenoxy]
-2-nitrobenzamide

N-[2-methyl-4-(phenylsulfonyl)
phenyl] 5-[2-chloro-4-(trifluoromethyl) phenoxy]-2-nitrobenzamide

N-(4-aminophenylsulfonyl)-N-
(methoxycarbonyl)
5-[2-chloro-4-(trifluoromethyl)
phenoxy]-2-nitrobenzamide

N-[(hydroxyimino) methyl]
5-[2-chloro-4-(trifluoromethyl)
phenoxy]-2-nitrobenzamide

5-[2-chloro-4-(trifluoromethyl) phenoxy]-2-nitrobenzoic acid hydrazide

5-[2-chloro-4-(trifluoromethyl) phenoxy]-2-nitrobenzoic acid methyl hydrazide

5-[2-chloro-4-(trifluoromethyl) phenoxy]-2-nitrobenzoyl (2-chloroacetyl) hydrazide

5-[2-chloro-4-(trifluoromethyl) phenoxy]-2-nitrobenzoyl 2-(carbox-amido) hydrazide

5-[2-chloro-4-(trifluoromethyl)-phenoxy]-2-nitrobenzoylcyanamide

5-[2-chloro-4-(trifluoromethyl)-phenoxy]-2-nitrobenzoylcyanamide sodium salt

5-[2-chloro-4-(trifluoromethyl) phenoxy]-2-nitrobenzoyl azide

aminomethyleneamino 5-[2-chloro-4-(trifluoromethyl) phenoxy]-2 nitrobenzoate

1-methylethylideneimino 5-[2-chloro -4-(trifluoromethyl) phenoxy]-2-nitrobenzoate

1-(methylthio) ethylideneamino 5[2-chloro-4-(trifluoromethyl) phenoxy]-2-nitrobenzoate

The compounds of this invention can be applied in various ways to achieve herbicidal action. They can be applied per se, as solids or in vaporized form, but are preferably applied as the toxic components in pesticidal compositions of the compound and a carrier. These compositions are preferably applied directly to the soil and often incorporated therewith. The compositions can be applied as granulars or dusts; as liquid sprays, or as gas-propelled sprays and can contain, in addition to a carrier, additives such as emulsifying agents, binding

agents, gases compressed to the liquid state, odorants, stabilizers, and the like. A wide variety of liquid and solid carriers can be used. Non-limiting examples of solid carriers include talc, bentonite, diatomaceous earth, pyrophyllite, fullers earth, gypsum, flours derived from cotton seeds and nut shells, and various natural and synthetic clays having a pH not exceeding about 9.5. Non-limiting examples of liquid carriers include water, organic solvents such as alcohols, ketones, light oils, and medium oils and vegetable oils such as cottonseed oil.

In practice, herbicidal application is measured in terms of pounds of herbicide applied per acre. The compounds of this invention are effective herbicides when applied in herbicidal amounts, i.e., at rates between about 0.2 and 10 kg. per hectare.

The pesticidal compositions comprising the active ingredient and the carrier may be supplied either as ready-to-use compositions or as concentrates. Concentrates generally contain a higher proportion of the active ingredient than the ready-to-use compositions and accordingly, require dilution prior to use. Both the ready-to-use compositions and the concentrates may be in liquid or solid form, i.e., with the active ingredient blended with a liquid or solid carrier, respectively.

The amount of active ingredient in the composition will depend, primarily, upon whether the composition is a concentrate or a ready-to-use composition. Concentrates will generally contain from 5 to 95% by weight, preferably 10 to 80% by weight, of the active ingredient. The concentration of active ingredient in the ready-to-use compositions will vary according to the method of application for the composition in question and to the desired application rate. In general, ready-to-use compositions will contain from 0.001 to 1, preferably 0.01 to 0.1 percent by weight of the active ingredient. Thus,

the compositions may contain from 0.001 to 95% by weight of active ingredient, the actual amount depending upon the nature of the composition and the method by which it shall be applied.

Concentrates may be either liquid or solid and are usually extendable with water to form emulsions or suspensions containing a smaller proportion of the active ingredient. Alternatively, liquid or solid concentrates may be extended with liquid or solid carriers to give the final ready-to-use composition. Liquid concentrates preferably comprise the active ingredient and an emulsifying agent dissolved in a liquid solvent, e.g. an organic solvent of the type mentioned above. The emulsifier is suitably an anionic, cationic or non-ionic emulsifier, e.g., sodium dodecylbenzenesulfonate or an ethylene oxide derivative of an alkyl phenol, a mercaptan, an amine or a carboxylic acid. Liquid concentrates may advantageously contain from 10 to 30 weight percent e.g. 25 weight percent of the active ingredient e.g. 1 kg. of active ingredient per 4 kg. of concentrate.

Wettable powders are another preferred form of concentrate and these suitably comprise the active ingredient, a finely-divided solid carrier and at least one surfactant to impart wettability or dispersability. Solid carriers which are suitable for preparing wettable powder formulations may be either organic or inorganic in nature. Suitable organic carriers are soybean, walnut, or wood flour or tobacco dust, and suitable inorganic ones are clays of the bentonite, kaolinite, of fuller's earth types; silicas such as diatomaceous earth; silicates such as talc, pyrophyllite, or alkaline earth silicates; and calcium and magnesium carbonates. The carrier may be a single substance or a mixture of finely divided solids. A surfactant or mixture of surfactants is generally present in an amount of 1 to 10 percent by weight of the wettable

powder formulation. Suitable dispersing agents are sodium formaldehydenapthalene sulfonate or sodium lignin sulfonate. Wetting agents include higher alkylaryl sulfonates ("higher alkyl" meaning alkyl of at least 8 carbon atoms) such as calcium dodecylbenzenesulfonate, alcohol sulfates, alkylphenoxyethoxyethoxyethyl sodium sulfonates, sodium dioctyl sulfosuccinate, and ethylene oxide adducts with fatty alcohols, fatty acids, or with higher alkyl-phenols, such as octylphenoxypolyethoxy-ethanol. Sticking or spreading agents may be included such as glycerol mannitan laurate or a condensate of polyglycerol and oleic acid modified with phthalic anhydride. The active ingredient content of the wettable powder may be in the range of 20 to 80% by weight; however, the preferred range of concentrations is 50% to 80%.

Dusts may be made by incorporating the active ingredient into a solid carrier, such as finely powdered clays, talc, silica, and synthetic silicates, alkaline earth carbonates, and diluents of natural origin such as tobacco dust or walnut shell flour. Granular formulations can be made from similar type solid carriers except that the particle size is larger in the range of 15 to 60 mesh (U.S. Standard Sieve Series). A small amount of dispersing agent may be incorporated in these solid formulations. The concentration of active ingredients in these dust or granular formulations may be in the range of 1 to 20% by weight. The solid carriers used in these formulations may be essentially inert or they may consist wholly or in part of fertilizing materials such as ammonium sulfate, or other ammonium salts, urea, calcium phosphates, potassium chloride or dried blood.

One particularly convenient method for making solid formulations is to treat the solid carrier with the active ingredients dissolved in a solvent and allow the solvent to evaporate off. This results in the carrier

which is usually in the form of finely divided particles, being impregnated with the active ingredients. Another method is to apply the mixture of active ingredients in the molten state or by spraying.

The concentration of the active ingredient in the final ready-to-use composition will depend not only upon the application rate desired (generally in the range of 0.2 to 10 kg. per hectare, as mentioned above), but also upon the application method which is to be used. Wettable powders and liquid concentrates are usually applied as aqueous sprays and applied at application rates varying from about 10 to 1500 liters per hectare. With ground equipment, the application rate will generally be in the range of 100 to 500 liters per hectare, whereas aerial spray equipment will generally apply 15 to 80 liters per hectare.

## Herbicidal Effectiveness

### Method of Propagating Test Species

Crop and weed species are planted in 8" x 10" disposable fiber flats containing potting soil to provide each flat with a 4" row of all test species. Crop species consist of field corn (CN), crabgrass (CG), cotton (CT), and soybeans (SB). The weed species consist of foxtail millet (FM), green foxtail (GF), velvetleaf (VL), cocklebur (CB), wild mustard (WM) and pigweed (PW).

Cotton, corn, soybean, and cocklebur plantings consist of 4 to 5 seeds per row depending upon species. The smaller seeded species (velvetleaf, wild mustard, pigweed, foxtail millet and green foxtail) are planted in an uncounted but sufficient number to provide a solid row of seedlings.

Plantings for the pre- and post-emergence portions of the test are identical as to seeding. The

initial watering until emergence is done from the top. The post- emergence phase is propagated in advance so as to provide plants of the proper stage of development at the time of treatment. Plantings for the pre-emergence phase are made not more than one day in advance of treatment.

The desired stage of development for treatment of the post-emergence broadleaf species (CT, SB, CB, VL, WM, PW) is the one true leaf or first trifoliate leaf stage. The desired stage for corn would be a height of 3-4", while a 2" height would be adequate for the grasses.

Method of Treatment

Spray applications are made with a handgun sprayer (aspirator type) simultaneously to one flat of established plants for the post-emergence phase and one newly seeded flat for the pre-emergence phase. The 10 lb./acre treatment rate consists of the uniform application of 116 milligrams of test compound to the combined area of the two flats (160 sq. inches). Application is made in a solvent mixture consisting of 40 ml acetone and 40 ml water and a surfactant concentration of 0.1%.

Following spray application, flats are returned to the greenhouse where watering of the post-emergence phase is done only by subirrigation. The pre-emergence phase is top watered by sprinkling until after test species have emerged. Subsequent watering is by subirrigation.

Two weeks after treatment, the pre- and post-emergence injury and control is rated on a 0-100% injury and control scale. Special physiological effects are rated as to intensity also at this time.

The herbicidal test data reported for compounds 1-81 was obtained at application rates of 10 lbs. down to 1/4 lb./acre. The following lists the metric equivalents for each rate.

| Application Rate | |
|---|---|
| US - lb./acre | Metric - kg/ha |
| 10.0 | 11.2 |
| 4.0 | 4.48 |
| 2.0 | 2.24 |
| 1.0 | 1.12 |
| 0.5 | 0.56 |
| 0.25 | 0.28 |

Test results are set forth in Table I(pre-emergence) and Table II (post-emergence).

## Table 1

| Cpd. No. | Dosage Lbs./Acre | Pre-Emergence CG | FM | GF | VL | CB | WM | PW | CT | CN | SB |
|---|---|---|---|---|---|---|---|---|---|---|---|
| 1 | 10 | - | 100 | 90 | 90 | 0 | 100 | 100 | 10 | 0 | 0 |
| | 4 | - | 100 | 90 | 20 | - | 100 | - | 0 | 0 | 0 |
| | 2 | - | 100 | 60 | 10 | - | 100 | 70 | 0 | 10 | 10 |
| | 1 | - | 90 | 90 | 30 | 0 | 100 | 100 | 20 | 0 | 10 |
| | 1/2 | - | 90 | 80 | 0 | 0 | 90 | 100 | 20 | 0 | 10 |
| | 1/4 | - | 90 | 70 | 30 | 0 | 90 | 80 | 0 | 10 | 0 |
| 2 | 10 | - | 20 | 20 | 10 | 0 | 70 | 90 | 0 | 0 | 0 |
| | 4 | - | - | - | - | - | - | - | - | - | - |
| | 2 | - | - | - | - | - | - | - | - | - | - |
| | 1 | - | - | - | - | - | - | - | - | - | - |
| | 1/2 | - | - | - | - | - | - | - | - | - | - |
| | 1/4 | - | - | - | - | - | - | - | - | - | - |
| 3 | 10 | - | 90 | 90 | 0 | 0 | 90 | 100 | 0 | 0 | 0 |
| | 4 | - | 100 | 90 | 30 | 20 | 90 | 100 | 10 | 0 | 0 |
| | 2 | - | 60 | 10 | 0 | 10 | 30 | 80 | - | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 4 | 10 | - | 100 | 90 | 90 | 20 | 100 | 100 | 10 | 0 | 10 |
| | 4 | - | 100 | 90 | 0 | - | 100 | - | 0 | 0 | 10 |
| | 2 | - | 90 | 40 | 0 | - | 70 | 60 | 0 | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |

| Cpd. No. | Dosage Lbs./ Acre | Pre-Emergence CG | FM | GF | VL | CB | WM | PW | CT | CN | SB |
|---|---|---|---|---|---|---|---|---|---|---|---|
| 5 | 4 | - | 90 | 90 | 70 | 30 | 100 | 100 | 60 | 0 | 0 |
| | 2 | - | 90 | 90 | 10 | 40 | 100 | 100 | 20 | 0 | 10 |
| | 1 | - | 70 | 10 | 0 | 0 | 70 | 100 | 0 | 0 | 10 |
| | 1/2 | - | - | - | - | - | - | - | - | - | - |
| | 1/4 | - | - | - | - | - | - | - | - | - | - |
| 6 | 4 | - | 100 | 90 | 70 | 20 | 100 | 100 | 0 | 40 | 0 |
| | 2 | - | 100 | 90 | 10 | 0 | 100 | 100 | 40 | 0 | 0 |
| | 1 | - | 90 | 60 | 0 | 40 | 90 | 90 | 30 | 0 | 0 |
| | 1/2 | - | 70 | 80 | 10 | 0 | 50 | 90 | 10 | 0 | 10 |
| | 1/4 | - | 20 | 0 | 10 | 10 | 30 | 0 | 0 | 20 | 10 |
| 7 | 4 | - | 0 | 0 | 0 | 0 | 100 | 100 | 40 | 0 | 0 |
| | 2 | - | 70 | 20 | 0 | 0 | 0 | 70 | 0 | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 8 | 10 | - | 0 | 0 | 0 | 0 | 0 | 0 | 0 | 30 | 0 |
| | 4 | - | - | - | - | - | - | - | - | - | - |
| | 2 | - | - | - | - | - | - | - | - | - | - |
| 9 | 4 | - | 0 | 0 | 0 | - | 10 | 60 | 10 | 0 | 0 |
| | 2 | - | - | - | - | - | - | - | - | - | - |
| | 1 | - | - | - | - | - | - | - | - | - | - |
| | 1/2 | - | 10 | 0 | 0 | 0 | 50 | 60 | 0 | 0 | 0 |
| | 1/4 | - | - | - | - | - | - | - | - | - | - |

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 10 | 4 | - | 10 | 10 | 10 | 0 | 0 | 0 | 10 | 10 | 10 |
| | 2 | - | - | - | - | - | - | - | - | - | - |
| 11 | 10 | - | 0 | 0 | 0 | 0 | 0 | 0 | 0 | 0 | 0 |
| | 4 | - | - | - | - | - | - | - | - | - | - |
| 12 | 4 | - | 100 | 90 | 90 | 60 | 100 | 100 | 40 | 10 | 0 |
| | 2 | - | 100 | 90 | 40 | 10 | 100 | 100 | 10 | 30 | 0 |
| | 1 | - | 100 | 90 | 0 | 0 | 100 | 100 | 0 | 0 | 0 |
| | 1/2 | - | 70 | 30 | 10 | 0 | 90 | 90 | 30 | 0 | 0 |
| | 1/4 | - | 20 | 20 | 0 | 0 | 20 | 60 | 60 | 60 | 30 |
| 13 | 10 | - | 90 | 90 | 100 | 80 | 100 | 100 | 90 | 10 | 10 |
| | 4 | - | 90 | 90 | 70 | 10 | 90 | 90 | 10 | 20 | 0 |
| | 2 | - | 90 | 70 | 30 | 0 | 90 | 90 | 0 | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 14 | 4 | - | 70 | 50 | 10 | 0 | 70 | 60 | 0 | 20 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 15 | 4 | - | 100 | 90 | 40 | 20 | 90 | 90 | 10 | 0 | 0 |
| | 2 | - | 90 | 90 | 10 | 0 | 90 | 90 | 0 | 0 | 0 |
| | 1 | - | 70 | 60 | 10 | 0 | 20 | 80 | 20 | 0 | 10 |
| | 1/2 | - | - | - | - | - | - | - | - | - | - |
| | 1/4 | - | - | - | - | - | - | - | - | - | - |

| Cpd. No. | Dosage Lbs./ Acre | Pre-Emergence | | | | | | | | | |
|---|---|---|---|---|---|---|---|---|---|---|---|
| | | CG | FM | GF | VL | CB | WM | PW | CT | CN | SB |
| 16 | 4 | - | 90 | 50 | 10 | 0 | 90 | 70 | 10 | 10 | 0 |
| | 2 | - | 80 | 10 | 10 | 0 | 70 | 0 | 0 | 0 | 10 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 17 | 4 | - | 100 | 90 | 40 | 0 | 100 | 100 | 0 | 10 | 0 |
| | 2 | - | 90 | 90 | 30 | 0 | 90 | 90 | 30 | 0 | 10 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 18 | 10 | - | 100 | 80 | 70 | 0 | 100 | 100 | 10 | 0 | 0 |
| | 2 | - | 100 | 40 | 20 | 10 | 100 | 100 | 10 | 0 | 10 |
| | 1/2 | - | 90 | 20 | 0 | 10 | 100 | 90 | 10 | 10 | 30 |
| | 1/4 | - | 10 | 10 | 40 | 10 | 80 | 60 | 10 | 0 | 0 |
| 19 | 10 | - | 100 | 100 | 80 | 60 | 100 | 100 | 30 | 60 | 0 |
| | 2 | - | 100 | 70 | 40 | 10 | 100 | 100 | 20 | 10 | 10 |
| | 1/2 | - | 100 | 60 | 0 | 0 | 100 | 60 | 10 | 0 | 0 |
| | 1/4 | - | 10 | 10 | 60 | 20 | 70 | 50 | 20 | 0 | 20 |
| 20 | 4 | - | 90 | 80 | 50 | 0 | 100 | 90 | 0 | 0 | 0 |
| | 2 | - | 80 | 20 | 10 | 20 | 90 | 20 | 0 | 0 | 10 |
| | 1 | - | 0 | 0 | 0 | 0 | 0 | 20 | 30 | 10 | 10 |
| | 1/2 | - | - | - | - | - | - | - | - | - | - |
| | 1/4 | - | - | - | - | - | - | - | - | - | - |
| 21 | 4 | - | 80 | 60 | 20 | 0 | 30 | 20 | 10 | 20 | 20 |
| | 2 | - | - | - | - | - | - | - | - | - | - |

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 22 | 4 | - | 90 | 80 | 20 | 0 | 90 | 90 | 0 | 10 | 0 |
| | 2 | - | 80 | 70 | 20 | 0 | 80 | 60 | 0 | 20 | 0 |
| | 1 | - | 90 | 20 | 10 | 20 | 30 | 10 | 40 | 10 | 50 |
| | 1/4 | - | - | - | - | - | - | - | - | - | - |
| 23 | 4 | - | 80 | 0 | 10 | 0 | 90 | 30 | 10 | 0 | 0 |
| | 2 | - | 70 | 10 | 30 | 0 | 70 | 10 | 0 | 0 | 0 |
| | 1/2 | - | - | - | - | - | - | - | - | - | - |
| | 1/4 | - | - | - | - | - | - | - | - | - | - |
| 24 | 4 | - | 90 | 70 | 60 | 10 | 90 | 90 | 0 | 0 | 10 |
| | 2 | - | 80 | 70 | 40 | 0 | 70 | 80 | 0 | 0 | 0 |
| | 1/2 | - | 0 | 10 | 0 | 0 | 0 | 10 | 0 | 0 | 0 |
| | 1/4 | - | - | - | - | - | - | - | - | - | - |
| 25 | 4 | - | 100 | 90 | 90 | 20 | 100 | 100 | 10 | 40 | 10 |
| | 2 | - | 90 | 90 | 80 | 40 | 100 | 100 | 40 | 10 | 20 |
| | 1 | - | 100 | 90 | 10 | 0 | 90 | 90 | 0 | 0 | 0 |
| | 1/2 | - | 90 | 70 | 0 | 0 | 90 | 70 | 10 | 0 | 10 |
| | 1/4 | - | 10 | 20 | 10 | 0 | 20 | 20 | 0 | 0 | 0 |
| 26 | 2 | - | 40 | 40 | 20 | 70 | 90 | 70 | 0 | 0 | 10 |
| | 1/2 | - | 10 | 0 | 10 | 0 | 90 | 40 | 0 | 0 | 0 |
| | 1/4 | - | - | - | - | - | - | - | - | - | - |
| 27 | 2 | - | 90 | 90 | 80 | 0 | 100 | 100 | 10 | 0 | 0 |
| | 1/2 | - | 70 | 50 | 40 | 0 | 90 | 100 | 0 | 0 | 0 |
| | 1/4 | - | 50 | 10 | 0 | 0 | 70 | 10 | 0 | 0 | 0 |

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 28 | 2 | - | 90 | 90 | 90 | 20 | 100 | 100 | 50 | 10 | 20 |
| | 1/2 | - | 90 | 90 | 20 | 0 | 100 | 100 | 10 | 0 | 0 |
| | 1/4 | - | 60 | 20 | 0 | - | 50 | 80 | 10 | 0 | 0 |
| 29 | 2 | - | 100 | 100 | 40 | 10 | 100 | 100 | 0 | 0 | 20 |
| | 1/2 | - | 100 | 90 | 0 | 0 | 100 | 90 | 10 | 0 | 0 |
| | 1/4 | - | 10 | 40 | 60 | 0 | 90 | 90 | 0 | 0 | 0 |
| 30 | 1/2 | 10 | 60 | - | 30 | - | 90 | 90 | - | - | - |
| | 1/4 | 10 | 40 | - | 10 | - | 10 | - | - | - | - |
| 31 | 2 | - | 60 | 50 | 10 | 0 | 80 | 90 | 30 | 20 | 0 |
| | 1/2 | - | 10 | 0 | 10 | 0 | 10 | 50 | 0 | 0 | 0 |
| | 1/4 | - | - | - | - | - | - | - | - | - | - |
| 32 | 2 | - | 90 | 70 | 0 | 0 | 100 | 100 | 10 | 0 | 10 |
| | 1/2 | - | 90 | 90 | 0 | 0 | 90 | 80 | 0 | 0 | 10 |
| | 1/4 | - | 30 | 10 | 0 | 0 | 10 | 0 | 0 | 0 | 0 |
| 33 | 2 | - | 100 | 90 | 50 | 0 | 90 | 90 | 10 | 10 | 40 |
| | 1/2 | - | 80 | 10 | 0 | 0 | 20 | 10 | 0 | 0 | 0 |
| | 1/4 | - | - | - | - | - | - | - | - | - | - |

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 34 | 2 | - | 100 | 100 | 10 | 30 | 90 | 90 | 30 | 10 | 0 |
| | 1/2 | - | 20 | 20 | 20 | 10 | 80 | 20 | 0 | 0 | 0 |
| | 1/4 | - | - | - | - | - | - | - | - | - | - |
| 36 | 2 | - | 50 | 20 | 40 | - | 20 | 90 | 10 | 20 | 30 |
| | 1/2 | - | 0 | 0 | 0 | - | 20 | 50 | 10 | 10 | 10 |
| | 1/4 | - | - | - | - | - | - | - | - | - | - |
| 37 | 2 | - | 60 | 60 | 0 | - | 80 | 20 | 0 | 0 | 0 |
| | 1/2 | - | 0 | 0 | 0 | - | 0 | 0 | 0 | 0 | 0 |
| | 1/4 | - | - | - | - | - | - | - | - | - | - |

| Cpd. No. | Dosage Lbs./ Acre | Pre-Emergence CG | FM | CF | VL | CB | WM | PW | CT | CN | SB |
|---|---|---|---|---|---|---|---|---|---|---|---|
| 38 | 2 | - | 100 | 90 | 50 | 10 | 100 | 100 | 60 | 0 | 0 |
| | 1/2 | - | 90 | 50 | 0 | 0 | 50 | 40 | 0 | 0 | 0 |
| | 1/4 | - | - | - | - | - | - | - | - | - | - |
| 39 | 2 | - | 90 | 90 | 70 | 20 | 100 | 100 | 10 | 20 | 0 |
| | 1/2 | - | 90 | 70 | 10 | 10 | 100 | 100 | 0 | 0 | 0 |
| 40 | 2 | - | 100 | 90 | 90 | 0 | 100 | 100 | 10 | 10 | 0 |
| | 1/2 | - | 90 | 70 | 20 | 0 | 100 | 90 | 10 | 0 | 0 |
| 41 | 2 | - | 100 | 90 | 40 | 0 | 100 | 90 | 10 | 0 | 0 |
| | 1/2 | - | 90 | 30 | 0 | 0 | 90 | 70 | 0 | 0 | 0 |
| | 1/4 | - | 10 | 0 | 0 | 0 | 10 | 10 | 0 | 0 | 0 |
| 42 | 2 | - | 100 | 100 | 90 | 70 | 100 | 100 | 90 | 20 | 30 |
| | 1/2 | - | 100 | 100 | 80 | 60 | 100 | 100 | 90 | 20 | 30 |
| | 1/4 | - | 80 | 90 | 10 | 0 | 60 | 90 | 10 | 0 | 10 |
| 43 | 1/2 | 90 | 90 | - | 50 | 0 | 90 | 100 | - | - | - |
| | 1/4 | 60 | 90 | - | 50 | - | 10 | - | - | - | - |
| 44 | 2 | - | 100 | 100 | 90 | 30 | 100 | 100 | 60 | 20 | 30 |
| | 1/2 | - | 90 | 90 | 80 | 10 | 100 | 100 | 20 | 10 | 20 |
| | 1/4 | - | 20 | 0 | 0 | 0 | 90 | 20 | 0 | 0 | 10 |

| Cpd. No. | Dosage Lbs./ Acre | Pre-Emergence CG | FM | GF | VL | CB | WM | PW | CT | CN | SB |
|---|---|---|---|---|---|---|---|---|---|---|---|
| 45 | 2 | - | 100 | 1( ) | 70 | 10 | 100 | 100 | 10 | 10 | 0 |
| | 1/2 | - | 90 | 80 | 10 | 0 | 100 | 100 | 0 | 0 | 0 |
| | 1/4 | - | 90 | 10 | 0 | 0 | 80 | 90 | 0 | 0 | 0 |
| 46 | 2 | - | 100 | 90 | 20 | 10 | 100 | 90 | 10 | 0 | 0 |
| | 1/2 | - | 90 | 90 | 10 | 30 | 90 | 70 | 10 | 0 | 0 |
| | 1/4 | - | 30 | 20 | 0 | 0 | 0 | 0 | 0 | 0 | 0 |
| 47 | 2 | - | 100 | 90 | 90 | 20 | 100 | 100 | 30 | 10 | 0 |
| | 1/2 | - | 100 | 90 | 20 | 10 | 100 | 100 | 10 | 0 | 0 |
| | 1/4 | - | 20 | 0 | 0 | 0 | 90 | 90 | 20 | 10 | 0 |
| 48 | 2 | - | 100 | 100 | 70 | 100 | 100 | 0 | 0 | 0 | 0 |
| | 1/2 | - | 100 | 90 | 30 | 0 | 90 | 60 | 40 | 0 | 0 |
| 49 | 2 | - | 90 | 90 | 60 | 0 | 90 | 100 | 0 | 0 | 0 |
| | 1/2 | - | 20 | 0 | 10 | 10 | 100 | 90 | 0 | 0 | 10 |
| 50 | 2 | - | 90 | 90 | 90 | 50 | 100 | 100 | 50 | 10 | 30 |
| | 1 | - | 90 | 90 | 20 | 0 | 100 | 90 | 0 | 0 | 0 |
| | 1/2 | - | 80 | 70 | 10 | 0 | 100 | 90 | 10 | 10 | 20 |
| | 1/4 | - | 90 | 70 | 20 | 10 | 90 | 80 | 0 | 0 | 0 |
| 51 | 10 | - | 100 | 100 | 100 | 50 | 100 | 100 | 10 | 30 | 0 |
| | 4 | - | 100 | 100 | 90 | 60 | 100 | 100 | 20 | 10 | 20 |
| | 2 | - | 90 | 90 | 70 | 10 | 100 | 100 | 20 | 0 | 10 |
| | 1 | - | 100 | 100 | 10 | 0 | 100 | 100 | 0 | 0 | 10 |
| | 1/2 | - | 90 | 50 | 0 | 10 | 70 | 40 | 0 | 0 | 0 |
| | 1/4 | - | - | - | - | - | - | - | - | - | - |

| Cpd. No. | Dosage Lbs./ Acre | Pre-Emergence CG | FM | GF | VL | CB | WM | PW | CT | CN | SB |
|---|---|---|---|---|---|---|---|---|---|---|---|
| 52 | 10 | 100 | 100 | - | 90 | - | 100 | - | - | - | - |
| | 1 | 60 | 70 | - | 60 | 0 | 90 | - | - | - | - |
| | 1/2 | 40 | 80 | - | 50 | 10 | 80 | - | - | - | - |
| | 1/4 | - | - | - | - | - | - | - | - | - | - |
| 53 | 10 | - | 90 | 90 | 50 | - | 100 | 100 | 50 | 10 | 0 |
| | 4 | - | 90 | 90 | 30 | - | 100 | 100 | 10 | 0 | 0 |
| | 1 | - | 90 | 60 | 0 | - | 90 | 90 | 0 | 0 | 0 |
| | 1/2 | - | 10 | 0 | 10 | - | 20 | 90 | 0 | 0 | 0 |
| | 1/4 | - | - | - | - | - | - | - | - | - | - |
| 54 | 4 | - | 0 | 0 | 0 | 0 | 0 | 0 | 0 | 0 | 0 |
| | 2 | - | - | - | - | - | - | - | - | - | - |
| | 1 | - | - | - | - | - | - | - | - | - | - |
| | 1/2 | - | - | - | - | - | - | - | - | - | - |
| | 1/4 | - | - | - | - | - | - | - | - | - | - |

| Cpd. No. | Dosage Lbs./Acre | Pre-Emergence CG | FM | GF | VL | CB | WM | PW | CT | CN | SB |
|---|---|---|---|---|---|---|---|---|---|---|---|
| 55 | 4 | - | 0 | 20 | 40 | 10 | 90 | 100 | 60 | 0 | 20 |
| | 2 | - | 0 | 0 | 0 | 0 | 0 | 0 | 0 | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 56 | 4 | - | 0 | 0 | 30 | - | 70 | 100 | 30 | 0 | 0 |
| | 2 | - | - | - | - | - | - | - | - | - | - |
| 57 | 4 | - | 10 | 0 | 0 | - | 70 | 90 | 0 | 0 | 0 |
| | 2 | - | - | - | - | - | - | - | - | - | - |
| | 1 | - | - | - | - | - | - | - | - | - | - |
| | 1/2 | - | - | - | - | - | - | - | - | - | - |
| | 1/4 | - | - | - | - | - | - | - | - | - | - |
| 58 | 4 | - | 30 | 0 | 0 | - | 10 | 100 | 0 | 0 | 0 |
| | 2 | - | - | - | - | - | - | - | - | - | - |
| | 1 | - | - | - | - | - | - | - | - | - | - |
| 59 | 10 | - | 0 | 0 | 0 | - | 0 | 100 | 0 | 0 | 0 |
| 60 | 10 | - | 0 | 0 | 10 | 0 | 10 | 70 | 0 | 0 | 0 |
| | 4 | - | - | - | - | - | - | - | - | - | - |
| 61 | 2 | - | 50 | 20 | 60 | 10 | 90 | 80 | 20 | 10 | 20 |
| | 1/2 | - | 0 | 0 | 0 | 0 | 50 | 0 | 0 | 0 | 0 |
| | 1/4 | - | - | - | - | - | - | - | - | - | - |

| Cpd. No. | Dosage Lbs./Acre | Pre-Emergence CG | FM | GF | VL | CB | WM | PW | CT | CN | SB |
|---|---|---|---|---|---|---|---|---|---|---|---|
| 62 | 4 | 90 | 100 | - | 80 | - | 100 | 100 | - | - | - |
| | 1 | 90 | 40 | - | 70 | - | 100 | 100 | - | - | - |
| | 1/2 | 40 | 0 | - | 10 | 0 | 80 | - | - | - | - |
| 63 | 4 | 90 | 90 | - | 100 | - | 100 | 100 | - | - | - |
| | 1 | 60 | 40 | - | 70 | - | 90 | 0 | - | - | - |
| | 1/2 | 20 | 0 | - | 20 | 0 | 10 | 0 | - | - | - |
| 64 | 4 | 90 | 90 | - | 90 | - | 100 | 100 | - | - | - |
| | 2 | 90 | 90 | - | 50 | - | 100 | 90 | - | - | - |
| | 1 | 20 | 0 | - | 40 | 0 | 90 | 0 | - | - | - |
| | 1/2 | 0 | 20 | - | 0 | 0 | 70 | 70 | - | - | - |
| 65 | 4 | 80 | 90 | - | 90 | - | 100 | - | - | - | - |
| | 2 | - | 30 | 70 | 20 | 0 | 80 | 100 | 0 | 0 | 0 |
| | 1 | - | 90 | 70 | 80 | 0 | 90 | 90 | 10 | 10 | 0 |
| | 1/2 | - | 10 | 10 | 50 | 0 | 100 | 100 | 0 | 0 | 0 |
| | 1/4 | - | 30 | 10 | 10 | 0 | 70 | 80 | 0 | 0 | 0 |
| 66 | 4 | 90 | 90 | - | 100 | - | 100 | 100 | - | - | - |
| | 1 | 70 | 40 | - | 70 | - | 100 | 100 | - | - | - |
| | 1/2 | 0 | 10 | - | 20 | 0 | 60 | 0 | - | - | - |
| 67 | 4 | 90 | 90 | - | 90 | - | 100 | 100 | - | - | - |
| | 2 | 60 | 70 | - | 60 | - | 90 | 90 | - | - | - |
| | 1 | 0 | 0 | - | 10 | 0 | 40 | 0 | - | - | - |
| | 1/2 | 0 | 0 | - | 40 | 10 | 90 | 20 | - | - | - |

| Cpd. No. | Dosage Lbs./ Acre | Pre-Emergence CG | FM | CF | VL | CB | WM | PW | CT | CN | SB |
|---|---|---|---|---|---|---|---|---|---|---|---|
| 68 | 10 | 100 | 90 | - | 100 | - | 100 | 100 | - | - | - |
| | 4 | - | 100 | 90 | 90 | 60 | 100 | 100 | 0 | 10 | 0 |
| | 2 | - | 90 | 90 | 90 | 10 | 100 | 100 | 20 | 20 | 20 |
| | 1 | - | 90 | 30 | 90 | 0 | 100 | 100 | 40 | 0 | 0 |
| | 1/2 | - | 70 | 80 | 70 | 50 | 100 | 100 | 10 | 0 | 0 |
| | 1/4 | - | 80 | 60 | 0 | 0 | 100 | 100 | 20 | 0 | - |
| 69 | 2 | - | 100 | 90 | 60 | 50 | 100 | 100 | 10 | 40 | 20 |
| | 1/2 | - | 100 | 90 | 10 | 10 | 100 | 100 | 0 | 0 | 10 |
| | 1/4 | - | 0 | 60 | 10 | 10 | 80 | 80 | 10 | 50 | 10 |
| 70 | 2 | - | 100 | 90 | 50 | 50 | 100 | 100 | 10 | 10 | 20 |
| | 1/2 | - | 100 | 90 | 10 | 30 | 100 | 100 | 20 | 0 | 10 |
| | 1/4 | - | 0 | 10 | 50 | 0 | 90 | 90 | 0 | 40 | 80 |
| 71 | 2 | - | 90 | 50 | 40 | 0 | 90 | 80 | 10 | 10 | 10 |
| | 1/2 | - | 10 | 10 | 0 | 0 | 10 | 90 | 10 | 0 | 10 |
| | 1/4 | - | - | - | - | - | - | - | - | - | - |
| 72 | 10 | - | 90 | 90 | - | 20 | 90 | 90 | - | 40 | 70 |
| | 2 | - | 90 | 90 | 70 | 0 | 100 | 100 | 10 | 20 | 0 |
| | 1 | - | 90 | 80 | 0 | 0 | 90 | 80 | 10 | 0 | 0 |
| | 1/2 | - | 20 | 0 | 10 | - | 90 | 90 | 20 | 20 | 10 |
| | 1/4 | - | 30 | 0 | 10 | - | 70 | 80 | 0 | 20 | 10 |
| 73 | 10 | - | 100 | 90 | 90 | 30 | 100 | 100 | 20 | 60 | 0 |
| | 4 | - | 100 | 90 | 80 | 60 | 100 | 90 | 50 | 70 | 10 |
| | 2 | - | 80 | 70 | 70 | 10 | 100 | 10 | 10 | 0 | 0 |
| | 1 | - | 90 | 60 | 10 | 0 | 90 | 60 | 20 | 0 | 10 |
| | 1/2 | - | 90 | 50 | 0 | 0 | 90 | 80 | 0 | 0 | 0 |

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 74 | 2 | - | 90 | 90 | 70 | 0 | 100 | 100 | 10 | 10 | 0 |
| | 1/2 | - | 90 | 70 | 0 | 0 | 90 | 50 | 10 | 0 | 0 |
| | 1/4 | - | 0 | 0 | 10 | 0 | 70 | 70 | 10 | 0 | 10 |
| 75 | 2 | - | 60 | 20 | 0 | 0 | 50 | 80 | 0 | 0 | 0 |
| | 1 | - | - | - | - | - | - | - | - | - | - |
| | 1/2 | - | - | - | - | - | - | - | - | - | - |
| | 1/4 | - | - | - | - | - | - | - | - | - | - |
| 76 | 2 | - | 80 | 40 | 80 | 0 | 70 | 70 | 20 | 60 | 10 |
| | 1/2 | - | 10 | 10 | 0 | 0 | 70 | 70 | 10 | 0 | 0 |
| | 1/4 | - | - | - | - | - | - | - | - | - | - |
| 77 | 2 | - | 100 | 90 | 10 | 0 | 100 | 90 | 0 | 0 | 0 |
| | 1/2 | - | 90 | 70 | 0 | 10 | 90 | 60 | 10 | 0 | 0 |
| 78 | 10 | 90 | 90 | - | 100 | - | 100 | - | - | - | - |
| | 4 | - | 90 | 90 | 90 | 0 | 100 | 100 | 0 | 0 | 0 |
| | 2 | - | 90 | 90 | 80 | 0 | 90 | 100 | 10 | 0 | 0 |
| | 1 | - | 40 | 60 | 60 | 10 | 100 | 100 | 0 | 0 | 0 |
| | 1/2 | - | 50 | 70 | 0 | - | 90 | 90 | 0 | 0 | 0 |
| | 1/4 | - | 80 | 50 | 20 | 0 | 90 | 60 | 10 | 30 | 0 |

FO 423

| Cpd. No. | Dosage Lbs./Acre | Pre-Emergence | | | | | | | | | |
|---|---|---|---|---|---|---|---|---|---|---|---|
| | | CG | FM | GF | VL | CB | WM | PW | CT | CN | SB |
| 79 | 10 | 70 | 90 | - | 50 | - | 90 | - | - | - | - |
| | 1/4 | 0 | 0 | - | 0 | - | 0 | - | - | - | - |
| 80 | 4 | - | 100 | 90 | 40 | 20 | 90 | 90 | 0 | 20 | 10 |
| | 2 | - | 90 | 80 | 10 | 10 | 90 | 10 | 10 | 10 | 10 |
| | 1 | - | 10 | 0 | 0 | 0 | 70 | 20 | 0 | 0 | 0 |
| | 1/2 | - | - | - | - | - | - | - | - | - | - |
| | 1/4 | - | - | - | - | - | - | - | - | - | - |
| 81 | 2 | - | 80 | 50 | 0 | 0 | 90 | 80 | 0 | 0 | 0 |
| | 1/2 | - | 10 | 0 | 0 | 0 | 10 | 60 | 0 | 0 | 0 |
| | 1/4 | - | - | - | - | - | - | - | - | - | - |

## Table II

| Cpd. No. | Dosage Lbs./ Acre | Post-Emergence CG | FM | GF | VL | CB | WM | PW | CT | CN | SB |
|---|---|---|---|---|---|---|---|---|---|---|---|
| 1 | 10 | - | 100 | 100 | 100 | 100 | 100 | 100 | 100 | 80 | 60 |
| | 4 | - | 90 | 90 | 100 | 100 | 100 | 100 | 90 | 60 | 50 |
| | 2 | - | 100 | 100 | 100 | - | 100 | - | 90 | 50 | 20 |
| | 1 | - | 100 | 90 | 100 | - | 100 | 100 | 90 | 30 | 50 |
| | 1/2 | - | 100 | 90 | 90 | 90 | 100 | 100 | 80 | 70 | 10 |
| | 1/4 | - | 50 | 50 | 40 | 80 | 100 | 100 | 60 | 50 | 40 |
| 2 | 10 | - | 90 | 90 | 90 | 80 | 90 | 100 | 90 | 10 | 10 |
| | 4 | - | 100 | 90 | 90 | 60 | 90 | 90 | 90 | 10 | 10 |
| | 2 | - | 90 | 90 | 90 | 80 | 100 | 100 | 90 | 20 | 20 |
| | 1 | - | 90 | 90 | 80 | 90 | 90 | 100 | 90 | 70 | 70 |
| | 1/2 | - | 80 | 70 | 60 | 90 | 100 | 100 | 90 | 80 | 30 |
| | 1/4 | - | 90 | 70 | 70 | 80 | 100 | 80 | 90 | 50 | 60 |
| 3 | 10 | - | 90 | 90 | 90 | 90 | 100 | 100 | 100 | 30 | 30 |
| | 4 | - | 100 | 100 | 90 | 90 | 100 | 100 | 90 | 30 | 10 |
| | 2 | - | 100 | 90 | 100 | 90 | 100 | 100 | 90 | 90 | 20 |
| | 1 | - | 90 | 90 | 90 | 90 | 100 | 100 | 80 | 70 | 60 |
| | 1/2 | - | 90 | 90 | 90 | 90 | 100 | 90 | 90 | 90 | 60 |
| | 1/4 | - | 90 | 80 | 90 | 80 | 100 | 100 | 90 | 30 | 10 |
| 4 | 10 | - | 100 | 100 | 100 | 100 | 100 | 100 | 100 | 90 | 50 |
| | 4 | - | 100 | 100 | 100 | - | 100 | - | 100 | 90 | 30 |
| | 2 | - | 90 | 90 | 100 | 100 | 100 | 100 | 100 | 20 | 20 |
| | 1 | - | 100 | 100 | 100 | - | 100 | 100 | 100 | 40 | 50 |
| | 1/2 | - | 100 | 90 | 90 | 90 | 100 | 100 | 100 | 70 | 10 |
| | 1/4 | - | 50 | 70 | 30 | 50 | 90 | 100 | 40 | 30 | 50 |

| Cpd. No. | Dosage Lbs./Acre | Post-Emergence CG | FM | GF | VL | CB | WM | PW | CT | CN | SB |
|---|---|---|---|---|---|---|---|---|---|---|---|
| 5 | 4 | - | 100 | 90 | 100 | 100 | 100 | 100 | 90 | 0 | 40 |
| | 2 | - | 90 | 90 | 100 | - | 100 | - | 90 | 10 | 20 |
| | 1 | - | 90 | 90 | 100 | 0 | 100 | 100 | 90 | 50 | 10 |
| | 1/2 | - | 80 | 70 | 90 | 80 | 100 | 100 | 90 | 20 | 20 |
| | 1/4 | - | 60 | 60 | 50 | 70 | 90 | 100 | 60 | 30 | 60 |
| 6 | 4 | - | 100 | 100 | 100 | 100 | 100 | 100 | 100 | 10 | 60 |
| | 2 | - | 100 | 100 | 100 | 100 | 100 | 100 | 90 | 80 | 40 |
| | 1 | - | 70 | 80 | 70 | 70 | 100 | 100 | 60 | 70 | 60 |
| | 1/2 | - | 90 | 90 | 90 | 90 | 100 | 90 | 80 | 80 | 20 |
| | 1/4 | - | 80 | 60 | 80 | 50 | 90 | 70 | 90 | 10 | 10 |
| 7 | 4 | - | 100 | 80 | 100 | 80 | 100 | 100 | 80 | 0 | 20 |
| | 2 | - | 90 | 30 | 100 | - | 90 | 100 | 90 | 10 | 20 |
| | 1 | - | 90 | 70 | 100 | - | 90 | 100 | 90 | - | 40 |
| | 1/2 | - | 90 | 90 | 100 | - | 90 | 100 | 90 | 10 | 50 |
| | 1/4 | - | 90 | 90 | 90 | 50 | 100 | 100 | 60 | 30 | 40 |
| 8 | 10 | - | 90 | 80 | 100 | 60 | 90 | 100 | 40 | 0 | 20 |
| | 4 | - | 60 | 30 | - | - | 70 | - | 70 | 20 | 0 |
| | 2 | - | 80 | 70 | 50 | - | 70 | 70 | 90 | 0 | 10 |
| 9 | 4 | - | 90 | 90 | 100 | 40 | 90 | 100 | 90 | 10 | 20 |
| | 2 | - | 90 | 90 | 90 | - | 90 | 100 | 90 | 10 | 30 |
| | 1 | - | 90 | 90 | 100 | 80 | 100 | 100 | 90 | 0 | 10 |
| | 1/2 | - | 100 | 90 | 90 | 70 | 100 | 100 | 80 | 10 | 10 |
| | 1/4 | - | 70 | 80 | 30 | 50 | 90 | 100 | 50 | 50 | 40 |

| Cpd. No. | Dosage Lbs./Acre | Post-Emergence | | | | | | | | | |
|---|---|---|---|---|---|---|---|---|---|---|---|
| | | CG | FM | GF | VL | CB | WM | PW | CT | CN | SB |
| 10 | 4 | - | 80 | 20 | 70 | 10 | 90 | 50 | 90 | 40 | 10 |
| | 2 | - | 50 | 50 | 70 | 0 | 90 | 70 | 90 | 70 | 30 |
| 11 | 10 | - | 90 | 90 | 70 | 30 | 80 | 100 | 40 | 0 | 10 |
| | 4 | - | 30 | 20 | 30 | 20 | 70 | 90 | 80 | 30 | 10 |
| 12 | 4 | - | 90 | 90 | 100 | 90 | 100 | 100 | 90 | 20 | 10 |
| | 2 | - | 100 | 100 | 90 | 100 | 100 | 100 | 100 | 70 | 40 |
| | 1 | - | 100 | 100 | 100 | 100 | 100 | 100 | 100 | 60 | 10 |
| | 1/2 | - | 100 | 100 | 90 | 80 | 100 | 100 | 80 | 80 | 60 |
| | 1/4 | - | 90 | 90 | 90 | 70 | 100 | 100 | 90 | 60 | 50 |
| 13 | 10 | - | 90 | 90 | 100 | 100 | 100 | 100 | 90 | 80 | 20 |
| | 4 | - | 100 | 100 | 100 | 100 | 100 | 100 | 100 | 90 | 90 |
| | 2 | - | 100 | 100 | 90 | 90 | 100 | 100 | 100 | 90 | 50 |
| | 1 | - | 90 | 90 | 90 | 70 | 100 | 100 | 90 | 70 | 50 |
| | 1/2 | - | 90 | 90 | 80 | 70 | 100 | 100 | 90 | 70 | 60 |
| | 1/4 | - | 90 | 80 | 80 | 80 | 100 | 90 | 90 | 60 | 60 |
| 14 | 4 | - | 90 | 90 | 90 | 80 | 100 | 100 | 90 | 30 | 20 |
| | 1 | - | 90 | 90 | 80 | 10 | 90 | 90 | 90 | 70 | 40 |
| | 1/2 | - | 90 | 90 | 70 | 70 | 90 | 90 | 80 | 70 | 20 |
| | 1/4 | - | 80 | 80 | 70 | 50 | 90 | 100 | 80 | 50 | 70 |
| 15 | 4 | - | 90 | 100 | 100 | 90 | 100 | 100 | 100 | 60 | 10 |
| | 2 | - | 90 | 90 | 90 | 90 | 100 | 90 | 90 | 70 | 30 |
| | 1 | - | 90 | 90 | 80 | 60 | 90 | 100 | 90 | 50 | 30 |
| | 1/2 | - | 90 | 90 | 90 | 90 | 100 | 100 | 90 | 50 | 30 |
| | 1/4 | - | 90 | 70 | 60 | 50 | 70 | 90 | 70 | 50 | 70 |

| Cpd. No. | Dosage Lbs./ Acre | Post-Emergence CG | FM | GF | VL | CB | WM | PW | CT | CN | SB |
|---|---|---|---|---|---|---|---|---|---|---|---|
| 16 | 4 | - | 90 | 90 | 60 | 50 | 100 | 100 | 90 | 50 | 10 |
| | 2 | - | 30 | 40 | 60 | 30 | 90 | 80 | 70 | 50 | 80 |
| | 1 | - | 70 | 60 | 70 | 70 | 90 | 90 | 90 | 60 | 70 |
| | 1/2 | - | 90 | 70 | 70 | 80 | 100 | 100 | 90 | 60 | 40 |
| | 1/4 | - | 60 | 40 | 60 | 30 | 90 | 100 | 60 | 50 | 50 |
| 17 | 4 | - | 100 | 100 | 100 | 90 | 100 | 100 | 90 | 70 | 30 |
| | 2 | - | 100 | 100 | 100 | 90 | 100 | 100 | 100 | 80 | 40 |
| | 1 | - | 90 | 90 | 80 | 60 | 90 | 100 | 70 | 70 | 40 |
| | 1/2 | - | 90 | 90 | 90 | 90 | 90 | 90 | 90 | 70 | 10 |
| | 1/4 | - | 80 | 80 | 80 | 50 | 90 | 60 | 90 | 40 | 10 |
| 18 | 10 | - | 100 | 100 | 100 | 90 | 100 | 100 | 90 | 90 | 90 |
| | 2 | - | 90 | 90 | 100 | 90 | 100 | 100 | 90 | 90 | 80 |
| | 1/2 | - | 90 | 90 | 100 | 70 | 100 | 100 | 80 | 70 | 60 |
| | 1/4 | - | 90 | 70 | 90 | 50 | 100 | 100 | 70 | 70 | 50 |
| 19 | 10 | - | 100 | 100 | 100 | 90 | 100 | 100 | 90 | 80 | 40 |
| | 2 | - | 90 | 90 | 100 | 100 | 100 | 100 | 90 | 60 | 40 |
| | 1/2 | - | 90 | 90 | 90 | 90 | 100 | 100 | 90 | 80 | 40 |
| | 1/4 | - | 90 | 70 | 70 | 40 | 100 | 100 | 50 | 60 | 20 |
| 20 | 4 | - | 90 | 90 | 90 | 80 | 100 | 100 | 90 | 40 | 20 |
| | 2 | - | 70 | 80 | 90 | 70 | 90 | 90 | 90 | 60 | 40 |
| | 1 | - | 80 | 60 | 70 | 70 | 90 | 80 | 90 | 50 | 20 |
| | 1/2 | - | 20 | 20 | 60 | 30 | 90 | 90 | 90 | 30 | 30 |
| | 1/4 | - | 50 | 40 | 40 | 40 | 100 | 50 | 50 | 40 | 50 |
| 21 | 4 | - | 80 | 70 | 90 | 40 | 90 | 90 | 90 | 50 | 20 |
| | 2 | - | 10 | 20 | 70 | 70 | 80 | 70 | 70 | 30 | 30 |

| Cpd. No. | Dosage Lbs./Acre | Post-Emergence | | | | | | | | | |
|---|---|---|---|---|---|---|---|---|---|---|---|
| | | CG | [illegible] | [illegible] | VL | CB | WM | PW | CT | CN | SB |
| 22 | 4 | - | 90 | 90 | 90 | 70 | 90 | 100 | 90 | 80 | 30 |
| | 2 | - | 80 | 80 | 90 | 20 | 90 | 100 | 90 | 80 | 50 |
| | 1 | - | 90 | 90 | 70 | 40 | 90 | - | 60 | 50 | 70 |
| | 1/4 | - | 50 | 60 | 10 | 10 | 80 | 80 | 50 | 10 | 10 |
| 23 | 4 | - | 90 | 90 | 80 | 60 | 90 | 100 | 90 | 80 | 80 |
| | 2 | - | 90 | 90 | 90 | 70 | 90 | - | 90 | 40 | 40 |
| | 1/2 | - | 20 | 20 | 20 | 10 | 90 | 80 | 80 | 10 | 10 |
| | 1/4 | - | 70 | 70 | 30 | 20 | 80 | 70 | 80 | 10 | 30 |
| 24 | 4 | - | 100 | 100 | 100 | 90 | 100 | 100 | 90 | 90 | 90 |
| | 2 | - | 100 | 100 | 100 | 80 | 100 | - | 90 | 50 | 70 |
| | 1/2 | - | 60 | 50 | 70 | 20 | 80 | 100 | 90 | 20 | 30 |
| | 1/4 | - | 70 | 80 | 70 | 50 | 90 | 90 | 90 | 10 | 40 |
| 25 | 4 | - | 100 | 100 | 100 | 100 | 100 | 100 | 100 | 90 | 80 |
| | 2 | - | - | - | - | - | - | - | - | - | - |
| | 1 | - | 100 | 100 | 100 | 90 | 100 | 90 | 100 | 90 | 70 |
| | 1/2 | - | 90 | 90 | 90 | 90 | 100 | 100 | 100 | 90 | 90 |
| | 1/4 | - | 80 | 80 | 90 | 80 | 90 | 90 | 90 | 40 | 30 |
| 26 | 2 | - | 50 | 70 | 100 | 90 | 100 | 100 | 90 | 30 | 60 |
| | 1/2 | - | 10 | 0 | 90 | 50 | 90 | 90 | 50 | 20 | 40 |
| | 1/4 | - | 20 | 10 | 60 | 80 | 90 | 100 | 50 | 10 | 50 |
| 27 | 2 | - | 100 | 100 | 100 | 100 | 100 | 100 | 90 | 70 | 90 |
| | 1/2 | - | 100 | 100 | 100 | 90 | 100 | 100 | 90 | 60 | 60 |
| | 1/4 | - | 70 | 60 | 90 | 70 | 100 | 60 | 90 | 50 | 80 |

| Cpd. No. | Dosage Lbs./Acre | Post-Emergence | | | | | | | | | |
|---|---|---|---|---|---|---|---|---|---|---|---|
| | | CG | FM | CF | VL | CB | WM | PW | CT | CN | SB |
| 28 | 2 | - | 100 | 90 | 100 | 100 | 100 | 100 | 100 | 90 | 90 |
| | 1/2 | - | 90 | 90 | 90 | 90 | 100 | 100 | 90 | 90 | 60 |
| | 1/4 | - | 90 | 90 | 90 | 80 | 100 | 100 | 100 | 70 | 80 |
| 29 | 2 | - | 100 | 100 | 100 | 90 | 100 | 100 | 100 | 60 | 90 |
| | 1/2 | - | 90 | 90 | 100 | 80 | 100 | 100 | 90 | 10 | 80 |
| | 1/4 | - | 90 | 90 | 100 | 70 | 100 | 100 | 90 | 50 | 80 |
| 30 | 1/2 | 20 | 100 | - | - | - | 100 | 100 | 90 | 80 | 60 |
| | 1/4 | 90 | 90 | - | 70 | 100 | 100 | 100 | 90 | 20 | 40 |
| 31 | 2 | - | 90 | 90 | 100 | 90 | 100 | 100 | 90 | 30 | 60 |
| | 1/2 | - | 90 | 90 | 100 | 70 | 90 | 100 | 90 | 60 | 30 |
| | 1/4 | - | 80 | 80 | 100 | 60 | 80 | 100 | 80 | 30 | 70 |
| 32 | 2 | - | 100 | 100 | 100 | 90 | 100 | 100 | 100 | 40 | 80 |
| | 1/2 | - | 100 | 100 | 100 | 80 | 100 | 100 | 100 | 20 | 80 |
| | 1/4 | - | 30 | 20 | 100 | 90 | 90 | 90 | 70 | 60 | 40 |
| 33 | 2 | - | 100 | 100 | 100 | 90 | 100 | 100 | 100 | 70 | 80 |
| | 1/2 | - | 90 | 90 | 100 | 70 | 100 | 100 | 100 | 70 | 80 |
| | 1/4 | - | 80 | 70 | 90 | 90 | 100 | 100 | 70 | 10 | 40 |

| Cpd. No. | Dosage Lbs./Acre | Post-Emergence | | | | | | | | | |
|---|---|---|---|---|---|---|---|---|---|---|---|
| | | CG | FM | GF | VL | CB | WM | PW | CT | CN | SB |
| 34 | 2 | - | 70 | 70 | 100 | 90 | 100 | 100 | 90 | 60 | 60 |
| | 1/2 | - | 60 | 60 | 100 | 90 | 100 | 100 | 80 | 40 | 40 |
| | 1/4 | - | 90 | 70 | 90 | 90 | 100 | 100 | 50 | 20 | 50 |
| 36 | 2 | - | 100 | 90 | 90 | 80 | 100 | 100 | 90 | 90 | 50 |
| | 1/2 | - | 90 | 90 | 90 | 30 | 90 | 100 | 90 | 70 | 60 |
| | 1/4 | - | 20 | 30 | 90 | - | 90 | 90 | 90 | 40 | 10 |
| 37 | 2 | - | 90 | 90 | 80 | 0 | 90 | 90 | 90 | 80 | 70 |
| | 1/2 | - | 90 | 90 | 90 | - | 90 | 90 | 90 | 40 | 40 |
| | 1/4 | - | 10 | 10 | 70 | - | 80 | 70 | 50 | 10 | 30 |

| Cpd. No. | Dosage Lbs./Acre | Post-Emergence CG | FM | CF | VL | CB | WM | PW | CT | CN | SB |
|---|---|---|---|---|---|---|---|---|---|---|---|
| 38 | 2 | - | 90 | 90 | 100 | 90 | 100 | 100 | 90 | 50 | 60 |
| | 1/2 | - | 90 | 90 | 100 | 90 | 100 | 100 | 60 | 10 | 50 |
| | 1/4 | - | 30 | 20 | 90 | 80 | 100 | 100 | 70 | 10 | 10 |
| 39 | 2 | - | 90 | 90 | 100 | 100 | 100 | 100 | 100 | 60 | 90 |
| | 1/2 | - | 90 | 90 | 90 | 90 | 100 | 100 | 100 | 40 | 70 |
| 40 | 2 | - | 100 | 100 | 100 | 100 | 100 | 100 | 100 | 90 | 90 |
| | 1/2 | - | 90 | 90 | 100 | 90 | 100 | 100 | 100 | 30 | 70 |
| 41 | 2 | - | 100 | 100 | 100 | 100 | 100 | 100 | 100 | 90 | 90 |
| | 1/2 | - | 90 | 90 | 100 | 90 | 100 | 90 | 90 | 30 | 80 |
| | 1/4 | - | 70 | 80 | 90 | 70 | 100 | 90 | 90 | 20 | 60 |
| 42 | 2 | - | 100 | 100 | 100 | 100 | 100 | 100 | 100 | 100 | 90 |
| | 1/2 | - | 100 | 100 | 90 | 90 | 100 | 100 | 100 | 90 | 90 |
| | 1/4 | - | 100 | 100 | 100 | 80 | 100 | 100 | 90 | 50 | 80 |
| 43 | 1/2 | 70 | 100 | - | 90 | 100 | 100 | 100 | 100 | 40 | 40 |
| | 1/4 | 100 | 100 | - | 90 | 100 | 100 | 100 | 70 | 40 | 30 |
| 44 | 2 | - | 100 | 100 | 100 | 100 | 100 | 100 | 100 | 90 | 90 |
| | 1/2 | - | 100 | 100 | 100 | 90 | 100 | 100 | 90 | 80 | 80 |
| | 1/4 | - | 90 | 90 | 90 | 70 | 100 | 100 | 90 | 60 | 60 |

| Cpd. No. | Dosage Lbs./Acre | Post-Emergence | | | | | | | | | |
|---|---|---|---|---|---|---|---|---|---|---|---|
| | | CG | FM | GF | VL | CB | WM | PW | CT | CN | SB |
| 45 | 2 | - | 100 | 100 | 100 | 100 | 100 | 100 | 100 | 100 | 90 |
| | 1/2 | - | 100 | 100 | 90 | 90 | 100 | 100 | 100 | 80 | 60 |
| | 1/4 | - | 100 | 100 | 90 | 90 | 100 | 100 | 90 | 50 | 60 |
| 46 | 2 | - | 100 | 100 | 100 | 90 | 100 | 100 | 100 | 90 | 90 |
| | 1/2 | - | 100 | 100 | 100 | 70 | 90 | 90 | 100 | 70 | 50 |
| | 1/4 | - | 90 | 90 | 90 | 30 | 90 | 90 | 90 | 50 | 50 |
| 47 | 2 | - | 100 | 100 | 100 | 100 | 100 | 100 | 100 | 90 | 80 |
| | 1/2 | - | 90 | 90 | 70 | 90 | 100 | 100 | 90 | 50 | 30 |
| | 1/4 | - | 90 | 90 | 60 | 20 | 100 | 100 | 80 | 20 | 60 |
| 48 | 2 | - | 100 | 100 | 100 | 90 | 100 | 100 | 100 | 90 | 80 |
| | 1/2 | - | 90 | 90 | 90 | 90 | 100 | 100 | 100 | 40 | 90 |
| 49 | 2 | - | 50 | 10 | 90 | 70 | 100 | 100 | 60 | 20 | 30 |
| | 1/2 | - | 10 | 20 | 70 | 30 | 100 | 100 | 90 | 40 | 30 |
| 50 | 2 | - | 90 | 90 | 60 | 90 | 100 | 100 | 90 | 70 | 40 |
| | 1 | - | 90 | 90 | 100 | 100 | 100 | 100 | 80 | 90 | 60 |
| | 1/2 | - | 60 | 80 | 90 | 70 | 100 | 100 | 80 | 0 | 10 |
| | 1/4 | - | 90 | 80 | 90 | 70 | 100 | 100 | 40 | 0 | 30 |
| 51 | 10 | - | 100 | 100 | 100 | 80 | 100 | 100 | 90 | 70 | 70 |
| | 4 | - | 100 | 100 | 100 | 100 | 100 | 100 | 90 | 90 | 70 |
| | 2 | - | 90 | 90 | 100 | 90 | 100 | 100 | 90 | 70 | 40 |
| | 1 | - | 100 | 100 | 100 | 100 | 100 | 100 | 80 | 90 | 80 |
| | 1/2 | - | 60 | 80 | 100 | 80 | 100 | 100 | 90 | 10 | 20 |
| | 1/4 | - | 100 | 90 | 100 | 70 | 100 | 90 | 50 | 0 | 40 |

| Cpd. No. | Dosage Lbs./Acre | Post-Emergence | | | | | | | | | |
|---|---|---|---|---|---|---|---|---|---|---|---|
| | | CG | FM | GF | VL | CB | WM | PW | CT | CN | SB |
| 52 | 10 | 70 | 90 | - | 100 | - | 100 | 100 | 80 | 30 | 30 |
| | 1 | 70 | 40 | - | 100 | 50 | 100 | 100 | 80 | 10 | 40 |
| | 1/2 | 30 | 20 | - | 70 | 20 | 90 | 100 | 40 | 10 | 10 |
| | 1/4 | 20 | 90 | - | 30 | 20 | 100 | 100 | 70 | 30 | 10 |
| 53 | 10 | 100 | 100 | - | 100 | 90 | 100 | 100 | 100 | 90 | 90 |
| | 4 | 100 | 90 | - | 80 | 80 | 100 | 100 | 100 | 70 | 80 |
| | 1 | 90 | 90 | - | 70 | 60 | 100 | 100 | 90 | 60 | 50 |
| | 1/2 | 40 | 40 | - | 20 | 0 | 100 | 90 | 80 | 20 | 50 |
| | 1/4 | 20 | 10 | - | 10 | - | 90 | 50 | 20 | 10 | 30 |
| 54 | 4 | - | 30 | 30 | 80 | 30 | 100 | 100 | 40 | 10 | 10 |
| | 2 | - | 20 | 20 | 10 | - | 90 | 90 | 20 | 10 | 10 |
| | 1 | - | 80 | 80 | 90 | 40 | 90 | 90 | 50 | 10 | 20 |
| | 1/2 | - | 60 | 50 | 10 | - | 90 | 90 | 30 | 10 | 10 |
| | 1/4 | - | 20 | 40 | 30 | 50 | 80 | 60 | 40 | 40 | 10 |

| Cpd. No. | Dosage Lbs./Acre | Post-Emergence CG | FM | GF | VL | CB | WM | PW | CT | CN | SB |
|---|---|---|---|---|---|---|---|---|---|---|---|
| 55 | 4 | - | 20 | 20 | 10 | 20 | 100 | 100 | - | 0 | 10 |
| | 2 | - | 30 | 30 | 0 | - | 100 | 80 | 20 | 0 | 80 |
| | 1 | - | 80 | 80 | 20 | 30 | 90 | 100 | 30 | 20 | 30 |
| | 1/2 | - | 80 | 80 | 10 | - | 90 | - | 80 | 0 | 10 |
| | 1/4 | - | 20 | 30 | 0 | 30 | 30 | 30 | 0 | 0 | 10 |
| 56 | 4 | - | 80 | 90 | 50 | 70 | 100 | 100 | 20 | 20 | 10 |
| | 2 | - | 30 | 10 | 30 | - | 60 | 80 | 70 | 0 | 10 |
| 57 | 4 | - | 60 | 80 | 40 | 30 | 90 | 100 | 70 | 0 | 10 |
| | 2 | - | 70 | 40 | 0 | - | 100 | 100 | 60 | 10 | 10 |
| | 1 | - | 60 | 50 | 0 | - | 90 | 90 | 60 | 40 | 30 |
| | 1/2 | - | 20 | 30 | 20 | - | 90 | 100 | 50 | 10 | 20 |
| | 1/4 | - | 20 | 20 | 10 | 30 | 60 | 80 | 50 | 10 | 10 |
| 58 | 4 | - | 40 | 70 | 0 | 40 | 90 | 100 | 30 | - | 20 |
| | 2 | - | 10 | 10 | 0 | - | 90 | 100 | 10 | 0 | 10 |
| | 1 | - | 50 | 50 | 30 | 20 | 70 | 100 | 30 | 20 | 30 |
| 59 | 10 | - | 0 | 10 | 0 | 0 | 0 | 80 | 0 | 0 | 0 |
| 60 | 10 | - | 80 | 60 | 10 | 70 | 90 | 100 | 30 | 10 | 10 |
| | 4 | - | 20 | 10 | 10 | 20 | 80 | 70 | 50 | 0 | 10 |
| 61 | 2 | - | 30 | 20 | 90 | 60 | 100 | 100 | 80 | 30 | 30 |
| | 1/2 | - | 20 | 30 | 80 | 50 | 100 | 100 | 70 | 20 | 30 |
| | 1/4 | - | 60 | 60 | 90 | 70 | 90 | 100 | 40 | 20 | 30 |

| Cpd. No. | Dosage Lbs./Acre | Post-Emergence | | | | | | | | | |
|---|---|---|---|---|---|---|---|---|---|---|---|
| | | CG | FM | GF | VL | CB | WM | PW | CT | CN | SB |
| 62 | 4 | 20 | 20 | - | 0 | 20 | 100 | - | 30 | 10 | 20 |
| | 1 | 0 | 10 | - | 10 | 10 | 60 | 70 | 30 | 0 | 30 |
| | 1/2 | 30 | 30 | - | 0 | 10 | 70 | 50 | 10 | 0 | 20 |
| 63 | 4 | 0 | 0 | - | 0 | 0 | 90 | - | 20 | 20 | 30 |
| | 1 | 0 | 30 | - | 20 | 10 | 50 | 80 | 10 | 10 | 20 |
| | 1/2 | 20 | 0 | - | 0 | 0 | 70 | 60 | 10 | 0 | 30 |
| 64 | 4 | 0 | 20 | - | 10 | 30 | 30 | 100 | 60 | 10 | 10 |
| | 2 | 0 | 10 | - | 20 | 10 | 100 | 90 | 70 | 10 | 10 |
| | 1 | 20 | 20 | - | 10 | 20 | 80 | 50 | 20 | 0 | 10 |
| | 1/2 | 10 | 10 | - | 10 | 10 | 80 | 60 | 0 | 10 | 20 |
| 65 | 4 | 10 | 20 | - | 30 | - | 100 | 100 | 70 | 20 | 40 |
| | 2 | - | 70 | 90 | 100 | 90 | 100 | 100 | 90 | 20 | 10 |
| | 1 | - | 90 | 90 | 100 | 90 | 100 | 100 | 80 | 30 | 40 |
| | 1/2 | - | 90 | 90 | 90 | - | 90 | 100 | 20 | 10 | 10 |
| | 1/4 | - | 50 | 40 | 70 | 70 | 90 | 100 | 80 | 30 | 40 |
| 66 | 4 | 0 | 40 | - | 10 | 20 | 90 | 100 | 20 | 40 | 30 |
| | 1 | 0 | 0 | - | 10 | 50 | 60 | 50 | 70 | 0 | 40 |
| | 1/2 | 10 | 10 | - | 20 | 40 | 40 | 50 | 10 | 0 | 40 |
| 67 | 4 | 20 | 20 | - | 10 | 60 | 40 | 100 | 50 | 40 | 50 |
| | 2 | 10 | 10 | - | 30 | 50 | 100 | 100 | 80 | 10 | 20 |
| | 1 | 20 | 20 | - | 0 | 10 | 20 | 100 | 20 | 10 | 40 |
| | 1/2 | 20 | 0 | - | 10 | 40 | 30 | 100 | 10 | 20 | 20 |

| Cpd. No. | Dosage Lbs./Acre | Post-Emergence CC | FM | GF | VL | CB | WM | PW | CT | CN | SB |
|---|---|---|---|---|---|---|---|---|---|---|---|
| 68 | 10 | 20 | 20 | - | 30 | - | 100 | 100 | 90 | 20 | 50 |
| | 4 | - | 100 | 100 | 100 | 100 | 100 | 100 | 90 | 90 | 60 |
| | 2 | - | 100 | 90 | 100 | 100 | 100 | 100 | 80 | 60 | 90 |
| | 1 | - | 30 | 50 | 100 | - | 90 | 100 | 90 | 20 | 30 |
| | 1/2 | - | 90 | 90 | 100 | - | 90 | 100 | 90 | 70 | 90 |
| | 1/4 | - | 50 | 40 | 80 | 80 | 90 | 100 | 70 | 30 | 60 |
| 69 | 2 | - | 100 | 90 | 100 | 80 | 100 | 100 | 90 | 80 | 70 |
| | 1/2 | - | 90 | 90 | 100 | 70 | 90 | 90 | 70 | 40 | 70 |
| | 1/4 | - | 50 | 40 | 100 | 60 | 90 | 90 | 90 | 50 | 60 |
| 70 | 2 | - | 90 | 80 | 100 | 70 | 100 | 100 | 90 | 70 | 60 |
| | 1/2 | - | 90 | 80 | 100 | 60 | 100 | 100 | 90 | 20 | 50 |
| | 1/4 | - | 90 | 70 | 100 | 50 | 90 | 90 | 80 | 20 | 40 |
| 71 | 2 | - | 40 | 60 | 100 | 90 | 100 | 100 | 90 | 20 | 60 |
| | 1/2 | - | 40 | 40 | 100 | 90 | 100 | 100 | 90 | 10 | 50 |
| | 1/4 | - | 20 | 10 | 60 | 80 | 90 | 100 | 50 | 10 | 50 |
| 72 | 10 | - | 100 | 100 | 100 | 100 | 100 | 100 | 100 | 90 | 80 |
| | 2 | - | 100 | 100 | 100 | 90 | 100 | 100 | 100 | 80 | 80 |
| | 1 | - | 100 | 100 | 100 | 90 | 100 | 100 | 100 | 80 | 80 |
| | 1/2 | - | 90 | 90 | 90 | 90 | 100 | 100 | 90 | 50 | 80 |
| | 1/4 | - | 90 | 90 | 90 | 80 | 100 | 100 | 100 | 30 | 80 |
| 73 | 10 | - | 100 | 100 | 100 | 70 | 100 | 100 | 90 | 80 | 50 |
| | 4 | - | 90 | 90 | 100 | 90 | 100 | 100 | 90 | 70 | 40 |
| | 2 | - | 30 | 50 | 70 | 40 | 100 | 100 | 50 | 10 | 30 |
| | 1 | - | 40 | 40 | 50 | 70 | 90 | 100 | 50 | 20 | 30 |
| | 1/2 | - | 90 | 90 | 80 | 70 | 100 | 100 | 70 | 20 | 20 |

| Cpd. No. | Dosage Lbs./ Acre | Post-Emergence CG | FM | GF | VL | CB | WM | PW | CT | CN | SB |
|---|---|---|---|---|---|---|---|---|---|---|---|
| 74 | 2 | - | 90 | 80 | 90 | 10 | 100 | 100 | 90 | 10 | 40 |
| | 1/2 | - | 90 | 80 | 90 | - | 100 | 90 | 90 | 20 | 30 |
| | 1/4 | - | 60 | 60 | 80 | 90 | 90 | 90 | 90 | 10 | 40 |
| 75 | 2 | - | 80 | 70 | 60 | 20 | 90 | 100 | 90 | 10 | 20 |
| | 1 | - | 90 | 80 | 80 | 40 | 100 | 70 | 70 | 20 | 20 |
| | 1/2 | - | 20 | 20 | 50 | 10 | 90 | 90 | 90 | 10 | 10 |
| | 1/4 | - | 10 | 10 | 70 | - | 60 | 20 | 50 | 0 | 20 |
| 76 | 2 | - | 20 | 10 | 40 | - | 90 | 100 | 90 | 10 | 50 |
| | 1/2 | - | 10 | 10 | 10 | - | 90 | 90 | 60 | 10 | 40 |
| | 1/4 | - | 10 | 10 | 80 | 30 | 80 | 70 | 40 | 10 | 10 |
| 77 | 2 | - | 90 | 90 | 100 | 90 | 100 | 100 | 50 | 20 | 30 |
| | 1/2 | - | 90 | 90 | 90 | 80 | 100 | 100 | 30 | 10 | 30 |
| 78 | 10 | 60 | 20 | - | 70 | - | 100 | 100 | 90 | 70 | 30 |
| | 4 | - | 80 | 90 | 100 | 90 | 100 | 100 | 90 | 0 | 10 |
| | 2 | - | 100 | 100 | 100 | 90 | 100 | 100 | 90 | 50 | 70 |
| | 1 | - | 90 | 90 | 100 | 60 | 100 | 100 | 40 | 80 | 40 |
| | 1/2 | - | 90 | 90 | 90 | 80 | 90 | - | 30 | 20 | 20 |
| | 1/4 | - | 40 | 80 | 40 | 50 | 80 | 100 | 40 | 40 | 40 |

| Cpd. No. | Dosage Lbs./Acre | Post-Emergence | | | | | | | | | |
|---|---|---|---|---|---|---|---|---|---|---|---|
| | | CG | FM | CF | VL | CB | WM | PW | CT | CN | SB |
| 79 | 10 | 50 | 60 | - | 80 | - | 100 | 100 | 80 | 10 | 20 |
| | 1/4 | 70 | 80 | - | 40 | 80 | 90 | 100 | 70 | 10 | 10 |
| 80 | 4 | - | 90 | 90 | 100 | 50 | 100 | 90 | 90 | 90 | 30 |
| | 2 | - | 70 | 50 | 90 | 50 | 90 | 60 | 90 | 60 | 90 |
| | 1 | - | 70 | 70 | 80 | 20 | 90 | 90 | 90 | 40 | 10 |
| | 1/2 | - | 30 | 30 | 70 | 50 | 90 | 90 | 90 | 60 | 20 |
| | 1/4 | - | 50 | 50 | 40 | 50 | 80 | 100 | 50 | 20 | 50 |
| 81 | 2 | - | 40 | 40 | 90 | 70 | 100 | 100 | 90 | 30 | 60 |
| | 1/2 | - | 30 | 30 | 100 | 50 | 100 | 100 | 90 | 10 | 40 |
| | 1/4 | - | 40 | 40 | 90 | 30 | 90 | 90 | 50 | 10 | 30 |

WHAT IS CLAIMED IS:

1. Herbicidal compounds of the formula

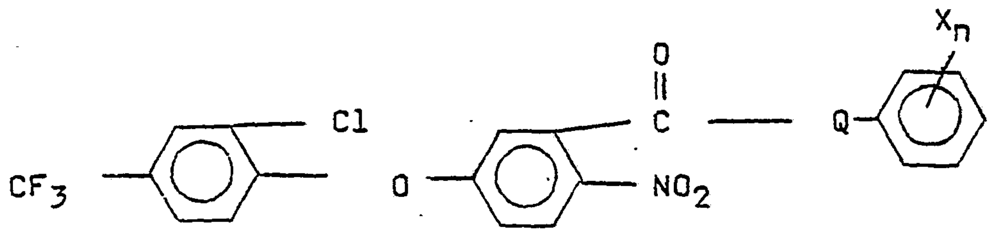

Where Q is O or S, n is an integer from 1 to 5, and X is selected from $NO_2$; lower alkyl of $C_{1-4}$; halogen; phenyl; COOR where R is lower alkyl of $C_{1-4}$, H or a cation selected from alkali metals, alkaline earth metals, ammonium and lower alkyl and di-lower alkyl ammonium; CN; lower alkyl thio of $C_{1-4}$; lower alkoxy of $C_{1-4}$; trifluoromethyl; and combinations thereof.

2. The compounds of Claim 1 wherein n is an integer from 1 to 3.

3. The compounds of Claim 2 wherein Q is O.

4. The compounds of Claim 3 wherein X is selected from the group consisting of $NO_2$, halogen, CN, COOR and $CF_3$ and combinations thereof.

5. Herbicidal compounds of the formula

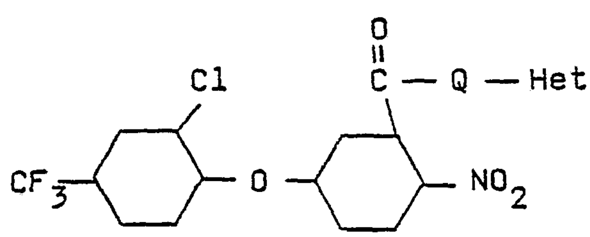

where Q is O or S and Het is a substituted or unsubstituted heterocyclic ring of 5 to 7 members comprising carbon in combination with one or more of sulfur, oxygen and nitrogen where a carbon atom on the ring is bound directly to Q.

6. The compounds of Claim 5 wherein Q is O.

7. The compounds of Claim 5 wherein Het is pyridine or substituted pyridine.

8. Herbicidal compounds of the formula

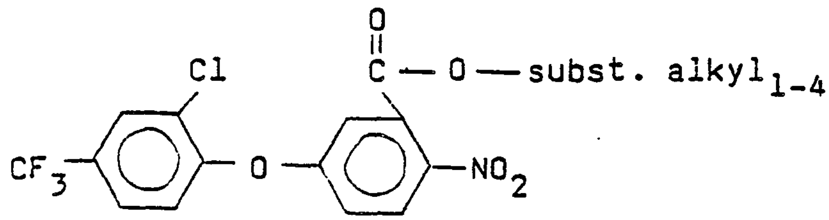

wherein the substituents on the straight or branched $C_{1-4}$ alkyl group are selected from cyano; phenyl; 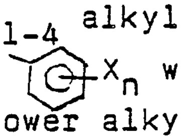 where n is 1 to 5 and X is selected from $NO_2$, lower alkyl of $C_{1-4}$, halogen, phenyl, phenoxy, CN, lower alkyl thio of $C_{1-4}$, lower alkoxy of $C_{1-4}$, trifluoromethyl and combinations thereof; 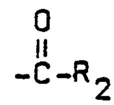 ; 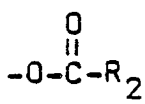 ; phenoxy; nitro; $SR_1$; $SOR_1$; $SO_2R_1$; where $R_1$ is an alkyl of 1 to 3 carbons and $R_2$ is an alkyl or alkenyl of up to 3 carbons and wherein one or more of said substituents can be attached to any one or more of the carbons on said straight or branched $C_{1-4}$ alkyl group.

9. The compounds of Claim 8 wherein the substituents on the alkyl group are selected from the group consisting of cyano, 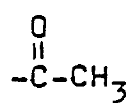 , $SCH_3$, $SOCH_3$, $SO_2CH_3$.

10. Herbicidal compounds of the formula

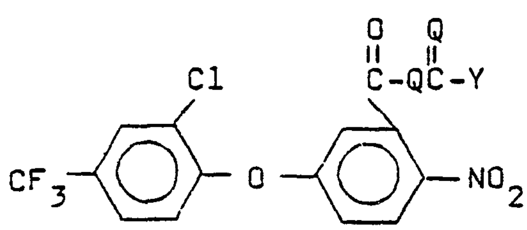

where Q is O or S and Y is a straight or branched alkoxy, alkylthio, alkenyloxy or alkenylthio of 1 to 6 carbons, a dialkylamino of 2 to 6 carbons, or a heterocyclic amine such as morpholine.

11. The compounds of Claim 10 wherein Y is a straight or branched alkoxy or alkylthio of 1 to 6 carbons.

12. Herbicidal compounds of the formula

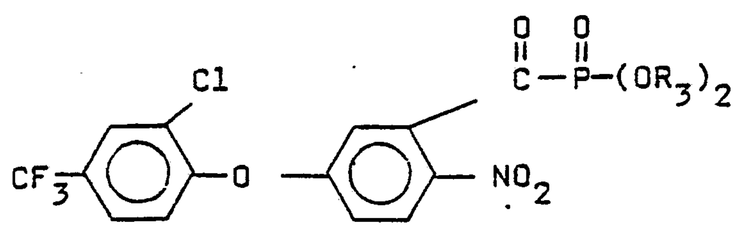

wherein $R_3$ is straight or branched alkyl of 1 to 6 carbons.

13. The compounds of Claim 12 wherein $R_3$ is a straight or branched alkyl of 1 to 3 carbons.

14. Herbicidal compounds of the formula

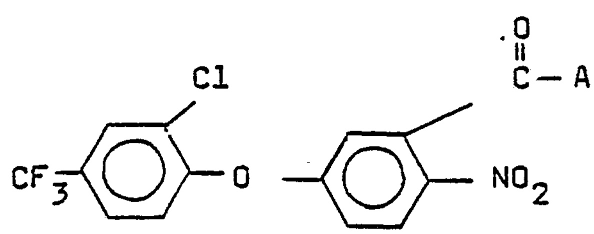

where A is an alkyl of 1 to 3 carbons or hydrogen.

15. The compounds of Claim 14 wherein A is hydrogen or methyl.

16. Herbicidal compounds of the formula

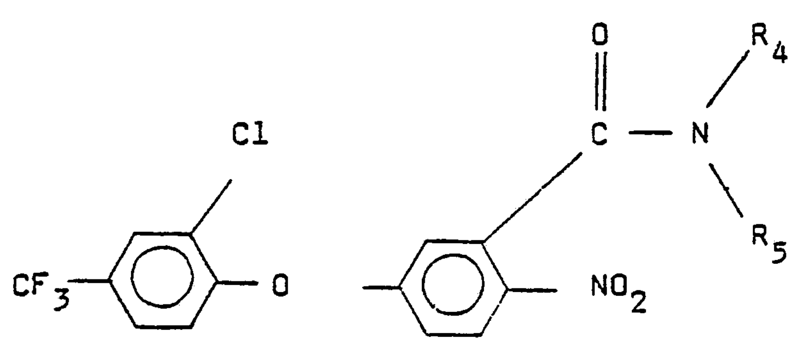

wherein $R_4$ is selected from hydrogen and straight or branched chain alkyl of 1-4 carbon; $R_5$ is selected from: phenyl; 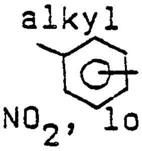$X_n$ where n is 1 to 5 and X is selected from $NO_2$, lower alkyl of $C_{1-4}$, halogen, phenyl, COOR where R is lower alkyl of $C_{1-4}$ or H or a cation, CN, lower alkyl thio of $C_{1-4}$, lower alkoxy of $C_{1-4}$, trifluoromethyl and combinations thereof; heterocyclicalkyl and heterocyclic, said heterocyclics of a 5 to 7 member ring comprising carbon in combination with one or more of sulfur, oxygen and nitrogen; hydroxyl, alkoxy of 1-3 carbons; $-R_6CO_2R_6$, where $R_6$ is an alkyl or alkenyl of up to 3 carbons; $-R_6CONH_2$; $R_6CONHR_2$ or $R_6CON(R_2)_2$; alkoxyalkyl where the alkoxy and alkyl may each contain a straight or branched $C_{1-3}$ alkyl chain; or where $R_4$ and $R_5$ together form a heterocyclic ring as hereinabove defined.

17. The compounds of Claim 16 where $R_5$ is 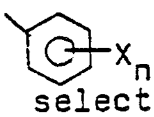$X_n$ wherein n is an integer of 1 to 3 and X is selected from the group consisting of $NO_2$, COOR and cyano and combinations thereof.

18. The compounds of Claim 16 wherein the heterocyclics for $R_5$ are selected from the group consisting of pyridine, tetrahydrofuran, thiophene.

19. The compounds of Claim 16 wherein $R_5$ is selected from the group consisting of alkoxy of 1-3 carbons, alkoxyalkyl where the alkoxy and alkyl moieties may each contain one to three carbons, and $R_6CONH_2$.

20. The compounds of Claim 16 wherein $R_4$ and $R_5$ together form a heterocyclic ring selected from the group consisting of five and six member rings comprising carbon in combinations with one or more of sulfur, oxygen and nitrogen and wherein said heterocyclic ring can be substituted by one or more substitutents selected from halogen, methyl and nitro and wherein said heterocyclic ring can be part of a bicyclic group, said second ring being phenyl.

21.

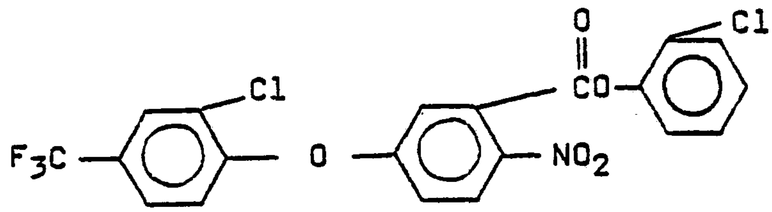

22.

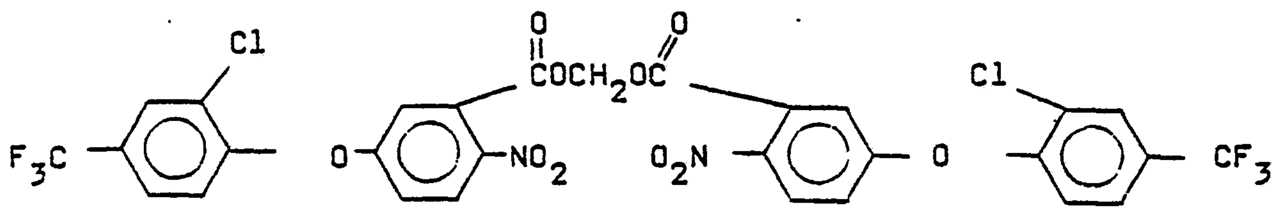

23.

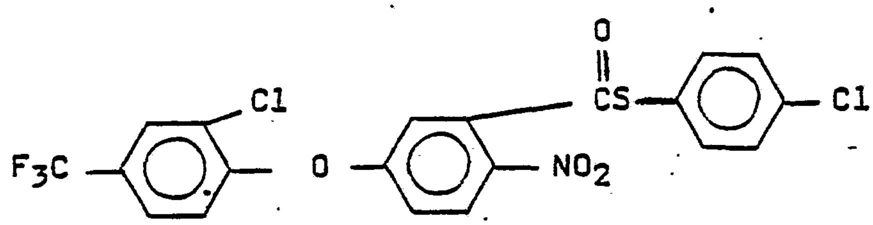

24.

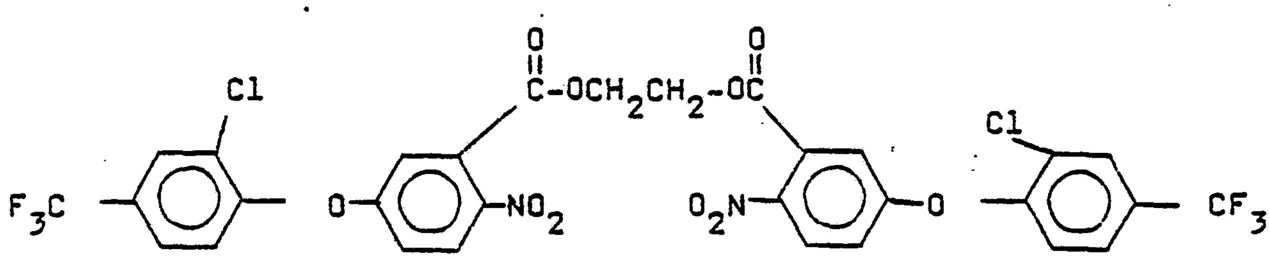

25. A herbicidal composition comprising a compound of any one of Claims 1 to 24 and a carrier therefor.

26. A method for combating undesirable herbs which comprises contacting them with a herbicidally effective amount of a compound according to any one of Claims 1 to 24.

European Patent Office

# EUROPEAN SEARCH REPORT

0034883

Application number

EP 81 30 0351

| DOCUMENTS CONSIDERED TO BE RELEVANT | | |
|---|---|---|
| Category | Citation of document with indication, where appropriate, of relevant passages | Relevant to claim |
| A | DE - A - 2 753 900 (ROHM AND HAAS)<br>* Pages 1,2; claim 1; page 3, claims 10 and 11 *<br>--- | 1 |
| A | US - A - 4 031 131 (W.O. JOHNSON)<br>* Page 1, abstract; column 1, lines 1-27 *<br>--------- | 1 |

CLASSIFICATION OF THE APPLICATION (Int. Cl.3)

C 07 C 79/46
A 01 N 37/10
C 07 C 153/11
A 01 N 35/04
C 07 D 213/30
C 07 D 333/46
C 07 C 121/38
C 07 C 149/18
C 07 F 9/40
C 07 C 79/36
C 07 C 103/30
A 01 N 43/34
A 01 N 43/10
A 01 N 37/18

TECHNICAL FIELDS SEARCHED (Int. Cl.3)

C 07 C 79/46

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 24.04.1981 | KINZINGER |